# EUROPEAN PATENT APPLICATION

(11) **EP 1 640 035 A1**
(43) Date of publication of application: **29.03.2006**
(21) Application number: 04734945.1
(22) Date of filing: 26.05.2004
(51) Int. Cl.: A61N 1/39, A61N 1/365

(54) **HEART TREATMENT APPARATUS AND HEART TREATMENT METHOD**

(30) Priority: 30.05.2003 JP 2003155531
(71) Applicant: TERUMO CORPORATION, Tokyo 151-0072 (JP)
(72) Inventor: FUKUI, Yoshihito, c/o Terumo Corporation, Ashigarakami-gun, Kanagawa 259-0151 (JP)
(74) Representative: Casalonga, Axel
(86) International application number: PCT/JP2004/007568
(87) International publication number: WO 2004/105870

(57) **Abstract**

There is disclosed heart treatment equipment in which it is possible to carry out controlling of a preventive treatment after an anti-tachyarrhthmia treatment in order to execute prevention and treatment of a fatal arrhythmia and the anti-tachyarrhythmia treatment is carried out by controlling tachyarrhythmia prevention means and tachyarrhythmia treatment means (tachyarrhythmia termination means) according to the result of detecting occurrence of tachyarrhythmia. In the anti-tachyarrhythmia treatment, there is provided with a structure for reducing or stopping the activation current with respect to the vagus nerve or a repression current with respect to the sympathetic nerve after a supply of an electroshock to the heart such as cardioversion, defibrillation or the like, so that it is possible to prevent deterioration of hemodynamics, recurrence of fatal arrhythmia and supraventicular arrhythmia such as atrial fibrillation or the like.

## Description

### TECHNICAL FIELD

The present invention relates to heart treatment equipment carrying out prevention and treatment of a fatal arrhythmia and more particularly relates to heart treatment equipment and a heart treatment method in which it is possible to carry out a control of a tachyarrhythmia prevention treatment method after an anti-tachyarrhythmia treatment.

### BACKGROUND ART

A sudden death especially caused by a heart disease is called a heart sudden death and the number thereof reaches about annual 50,000 people within the country. The direct cause of the heart sudden death is a generation of the ventricular tachycardia which causes a failure of the hemodynamics or the ventricular fibrillation and which is called a fatal arrhythmia. In a ventricular tachycardia, the ventricle beats convulsively and abnormally rapidly and in a ventricular fibrillation, each of the muscle fibers which constitute the ventricle starts excitement disorderedly and will be in a condition of only trembling little by little as a whole ventricle. When a fatal arrhythmia occurs, the pumping function of the heart lowers or disappears and it becomes impossible to send out the required blood to the whole body, so that an atypical absence is caused in accordance with the decrease of the cerebral blood flow and a death might be caused unless an immediately appropriate treatment is conducted.

For a patient having a risk of such a heart sudden death, a treatment which implants an implantable cardiac defibrillator (ICD) is carried out. When an occurrence of a ventricular tachycardia or a ventricular fibrillation is detected, the ICD gives an electroshock to the heart such that a defibrillation is carried out. However, the ICD is medical treatment equipment which is to say a countermeasure therapy operating after an abnormality occurs in the heart and in addition, there is a fear that it may give a large load to the heart tissue caused by the defibrillation.

Now, the heart activity is put under the control of an autonomic nerve system and the autonomic nerve system has a sympathetic nerve system and a parasympathetic nerve system where the parasympathetic nerve system of the heart is a vagus nerve. The heart activity (heart rate and cardiac output) increases when the activity of the sympathetic nerve increases (under tension) and the heart activity (heart rate) decreases when the activity of the vagus nerve increases. The activities of the sympathetic nerve and the vagus nerve are usually antagonistic each other, but when the sympathetic nerve tension is accentuated excessively and its balance becomes off-balanced, arrhythmia becomes easy to occur caused by accentuation of automaticity or shortening of a refractory period in a heart muscle or in an excitation conducting system and particularly for a patient of an organic heart disease such as myocardial infarction or cardiomyopathy, a possibility of a fatal arrhythmia occurrence is to be heightened by a combination of organic lesion of the heart and accentuation of a sympathetic nerve tension.

As a method for preventing occurrence (generation) of such a fatal arrhythmia, there has been attempted a method in which an excessive sympathetic nerve tension is blocked directly from being transmitted to the heart by stimulating the sympathetic nerve electrically, a method in which the sympathetic nerve tension is repressed antagonistic by stimulating the vagus nerve electrically, or a trial in which a lesion portion which caused an organic change is restructured by stimulating a nerve growth in a sinus node or a ventricle muscle after applying stimulation of a level which does not increase the heart rate and the blood pressure to a ganglion stellatum which is a sympathetic nerve.

There was already proposed in the past, based on such a principle, effective heart treatment equipment particularly for defibrillation in which necessary energy is low with respect to tachyarrhythmia prevention and acute treatment by a nerve stimulation (for example, see Patent Reference 1). This heart treatment equipment supplies a preventative activation current to the vagus nerve and at the same time supplies a repression current to the sympathetic nerve, and the vagus nerve is lightly activated for 5 seconds immediately after a chronic fibrillation or other dangerous tachyarrhythmia is detected. Then, the medical treatment will be finished when detecting a return to a usual condition and when it is desirable for continuously detecting an abnormal condition relating to the heart, the treatment is continued. Then, if it is advantageous to block transmission (conduction) of a sympathetic nerve excitement from the brain, this block is carried out additionally in the ganglion stellatum for a few seconds. Nevertheless, in a case when an abnormal state of the heart is detected, an electroshock is supplied by a conventional method.

There was also proposed a method and equipment in which an electric stimulation is applied to the right ganglion stellatum in order to prevent a fatal arrhythmia (for example, see Patent Reference 2). Equipment for preventing a fatal arrhythmia described in the Patent Reference 2 is equipment in which a risk of arrhythmia occurrence is to be reduced by applying a stimulation of a level which does not increase the heart rate and the blood pressure to the right ganglion stellatum, wherein nerve stimulation energy is adjusted to be under a threshold of a nerve stimulation which increases the heart rate or the blood pressure and nevertheless, in a case when arrhythmia occurrence (generation) is detected, a corresponding arrhythmia termination treatment is to be supplied.
[Patent Reference 1] Jap. laid-open patent publication H8-38625
[Patent Reference 2] Specification of USP6,487,450

However, the heart treatment equipment described in the Patent Reference 1 is not provided with a structure for reducing or stopping the activation current with respect to the vagus nerve or a repression current with respect to the sympathetic nerve after supplying an electroshock to the heart, so that there was a problem with deterioration of hemodynamics, recurrence of a fatal arrhythmia, induction of supraventricular arrhythmia such as atrial fibrillation or the like and the like.

Also, the arrhythmia treatment equipment described in the Patent Reference 2 is not provided with a control structure for reducing the nerve stimulation energy or for stopping the nerve stimulation subsequently to the arrhythmia termination treatment, so that it is not enough to solve a problem with deterioration of hemodynamics, recurrence of a fatal arrhythmia, induction of supraventricular arrhythmia such as atrial fibrillation or the like and the like similarly as the heart treatment equipment described in the Patent Reference 1.

### DISCLOSURE OF THE INVENTION

Taking a fact that the heart is in an unstable state after an anti-tachyarrhythmia treatment into consideration and in view of the aforesaid problem with deterioration of hemodynamics, recurrence of a fatal arrhythmia and induction of supraventricular arrhythmia such as atrial fibrillation or the like if a preventive therapy which affects suppressively with respect to the heart is applied under such a situation, the present invention has an object to offer heart treatment equipment and a heart treatment method in which it is possible to deactivate the tachyarrhythmia prevention treatment or to shift to a mode of a small nerve stimulation energy in response to tachyarrhythmia detection or confirmation of tachyarrhythmia continuation.

In order to solve aforesaid problems and to accomplish objects of the present invention, the heart treatment equipment of the present invention includes tachyarrhythmia prevention means for preventing occurrence of tachyarrhythmia; tachyarrhythmia detection means for detecting the occurrence of tachyarrhythmia; tachyarrhythmia termination means supplying anti-tachyarrhythmia treatment for terminating the tachyarrhythmia in response to the tachyarrhythmia detection means; and control means for connecting the tachyarrhythmia prevention means and the tachyarrhythmia detection means, wherein the control means executes a control for deactvating the tachyarrhythmia prevention means in its operation in response to the tachyarrhythmia detection means.

Also, according to a feature of the heart treatment equipment of the present invention, there are provided with tachyarrhythmia prevention means for preventing occurrence of tachyarrhythmia; tachyarrhythmia detection means for detecting the occurrence of tachyarrhythmia; tachyarrhythmia confirmation means for confirming that the tachyarrhythmia continues in response to the tachyarrhythmia detection means; tachyarrhythmia termination means supplying anti-tachyarrhythmia treatment for terminating the tachyarrhythmia in response to the tachyarrhythmia confirmation means; and control means for connecting the tachyarrhythmia prevention means and the tachyarrhythmia confirmation means, wherein the control means executes a control for deactivating the tachyarrhythmia prevention means in its operation in response to the tachyarrhythmia confirmation means.

According to a preferable mode of the present invention, it has a feature for the tachyarrhythmia prevention means such that nerve stimulation means for stimulating a sympathetic nerve(ganglion stellatum) or nerve stimulation means for stimulating a parasympathetic nerve (vagus nerve) is included and particularly, the stimulation of the sympathetic nerve is a stimulation having a level which does not increase the heart rate and the blood pressure or is a stimulation which blocks transmission of the sympathetic nerve excitement and also, the stimulation of the parasympathetic nerve is a stimulation which activates the parasympathetic nerve excitement.

Also, according to a preferable mode of the present invention, it has a feature for an anti-tachyarrhythmia treatment which the tachyarrhythmia termination means supplies such that at least one of anti-tachyarrhythmia pacing, cardioversion and defibrillation is included. Then, further, according to a preferable mode of the present invention, there are provided with bradycardia detection means for detecting occurrence of bradycardia and bradycardia treatment means for generating a heart stimulation pulse in response to the bradycardia detection means.

Further, according to a preferable mode of the present invention, it has a feature such that timing means for clocking a predetermined time is included and the timing means is to set a clocking time in conformity with the anti-tachyarrhythmia treatment in response to the supply of the anti-tachyarrhythmia treatment by means of the tachyarrhythmia termination means.

Also, according to a preferable mode of the present invention, it has a feature such that there is provided with tachyarrhythmia termination confirmation means for confirming whether or not the tachyarrhythmia is terminated by the tachyarrhythmia termination means and a tachyarrhythmia prevention treatment is carried out by the control means after the timing means carried out clocking of a predetermined time in response to aforesaid tachyarrhythmia termination confirmation means.

Also, according to a preferable mode of the present invention, it has a feature such that there is provided with tachyarrhythmia termination confirmation means for confirming whether or not tachyarrhythmia terminated by the tachyarrhythmia termination means and tachyarrhythmia prevention treatment is to be carried out by the control means after clocking a time period in conformity with a supplied anti-tachyarrhythmia treatment in response to the tachyarrhythmia termination confirmation means or after the measured heart rate exceeds a predetermined threshold.

Also, the present invention has a feature such that there are provided with tachyarrhythmia prevention means for preventing occurrence of tachyarrhythmia by a nerve stimulation; tachyarrhythmia detection means for detecting the occurrence of tachyarrhythmia; tachyarrhythmia termination means supplying anti-tachyarrhythmia treatment for terminating the tachyarrhythmia in response to tachyarrhythmia confirmation means; and control means for connecting the tachyarrhythmia prevention means and the tachyarrhythmia confirmation means, wherein the nerve stimulation by the tachyarrhythmia prevention means operates at least in a first operation mode and a second operation mode and at the same time, nerve stimulation energy of the second operation mode is set to be lower than nerve stimulation energy of the first operation mode such that the control means is to operate the tachyarrhythmia prevention means in the second operation mode in response to the tachyarrhythmia detection means.

Also, the present invention has a feature such that there are provided with tachyarrhythmia prevention means for preventing occurrence of tachyarrhythmia by a nerve stimulation; tachyarrhythmia detection means for detecting the occurrence of tachyarrhythmia; tachyarrhythmia confirmation means for confirming that aforesaid tachyarrhythmia continues in response to the tachyarrhythmia detection means; tachyarrhythmia termination means supplying anti-tachyarrhythmia treatment for terminating aforesaid tachyarrhythmia in response to tachyarrhythmia confirmation means; and control means for connecting the tachyarrhythmia prevention means and aforesaid tachyarrhythmia confirmation means, wherein the nerve stimulation by the tachyarrhythmia prevention means operates at least in a first operation mode and a second operation mode and at the same time, nerve stimulation energy of the second operation mode is set to be lower than nerve stimulation energy of the first operation mode such that the control means is to operate the tachyarrhythmia prevention means in the second operation mode in response to the tachyarrhythmia detection means.

It should be noted in connection with the technical feature in the above-mentioned heart treatment equipment that the present invention is offered as an invention of a heart treatment method which has similar features.

According to the present invention, there is provided with a structure for reducing or stopping activation current with respect to a vagus nerve or repression current with respect to a sympathetic nerve after an electroshock supply to the heart such as cardioversion, defibrillation or the like, so that it is possible to prevent deterioration of hemodynamics, recurrence of fatal arrhythmia and induction of supraventricular arrhythmia such as atrial fibrillation or the like. Also, tachyarrhythmia prevention treatment is canceled or is shifted to a mode having a smaller nerve stimulation energy in response to tachyarrhythmia detection or continuation confirmation of tachyarrhythmia, so that it is possible for a patient having a risk of heart sudden death to receive a moderate preventive treatment of fatal arrhythmia such as tachyarrhytmia or the like.

### BRIEF DESCRIPTION OF DRAWINGS

FIG. 1 is a block diagram showing a constitutional example of a first exemplified embodiment according to heart treatment equipment of the present invention;
FIG. 2 is an arrangement diagram of an electrode lead and a stimulation electrode to a heart, which are used in the heart treatment equipment of the present invention;
FIG. 3 a flow diagram for explaining operation of the first exemplified embodiment according to the present invention;
FIG. 4 a flow diagram also for explaining operation of the first exemplified embodiment according to the present invention;
FIG. 5 is a block diagram showing a constitutional example of a second exemplified embodiment according to the heart treatment equipment of the present invention;
FIG. 6 a flow diagram for explaining operation of the second exemplified embodiment according to the present invention;
FIG. 7 a flow diagram also for explaining operation of the second exemplified embodiment according to the present invention;
FIG. 8 is a block diagram showing a constitutional example of a third exemplified embodiment according to the heart treatment equipment of the present invention;
FIG. 9 a flow diagram for explaining operation of the third exemplified embodiment according to the present invention;
FIG. 10 a flow diagram also for explaining operation of the third exemplified embodiment according to the present invention;
FIG. 11 is a block diagram showing a constitutional example of a fourth exemplified embodiment according to the heart treatment equipment of the present invention;
FIG. 12 a flow diagram for explaining operation of the fourth exemplified embodiment according to the present invention;
FIG. 13 a flow diagram also for explaining operation of the fourth exemplified embodiment according to the present invention;
FIG. 14 is a block diagram showing a constitutional example of a fifth exemplified embodiment according to the heart treatment equipment of the present invention;
FIG. 15 a flow diagram for explaining operation of the fifth exemplified embodiment according to the present invention; and
FIG. 16 is a flow diagram also for explaining operation of the fifth exemplified embodiment according to the present invention.

### BEST MODE FOR CARRYING OUT THE INVENTION

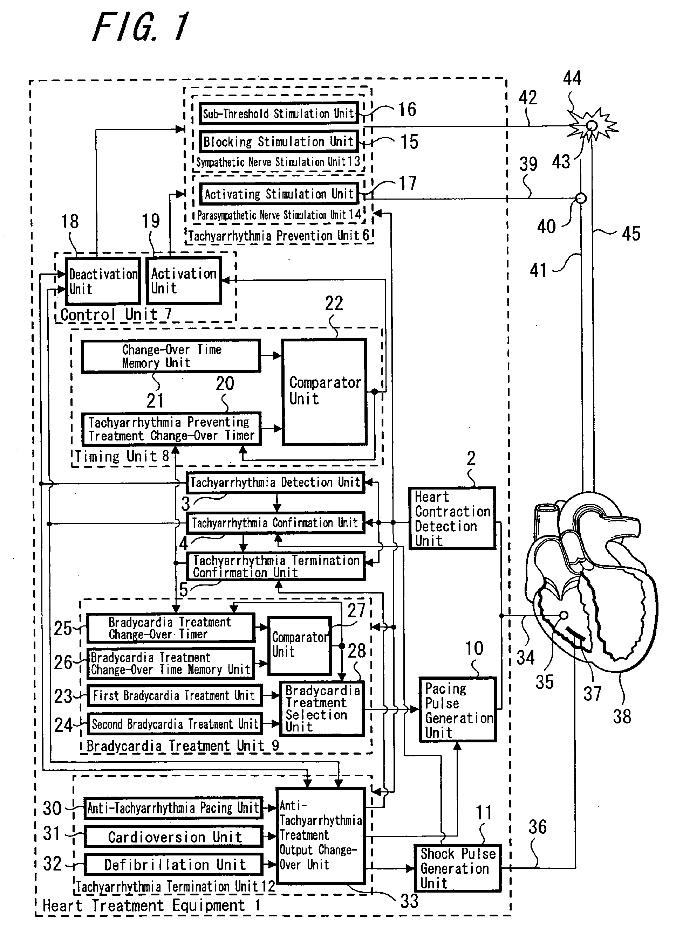

Hereinafter, a first exemplified embodiment of the heart treatment equipment according to the present invention will be explained with reference to the block diagram shown in FIG. 1.

Heart treatment equipment 1 of the present invention is constituted by a heart contraction detection unit 2 for detecting contraction of a heart 38, a tachyarrhythmia detection unit 3 for observing an output of the heart contraction detection unit 2 full-time and for detecting tachyarrhythmia, a tachyarrhythmia confirmation unit 4 for confirming continuous detection of the tachyarrhythmia, a tachyarrhythmia termination confirmation unit 5 for confirming that the tachyarrhythmia terminated by an anti-tachyarrhythmia treatment, a tachyarrhythmia prevention unit 6 for preventing the tachyarrhythmia by stimulating a vagus nerve 41 or a ganglion stellatum 44 of a sympathetic nerve 45, a control unit 7 for carrying out a control for deactivating or activating the operation of the tachyarrhythmia prevention unit 6, a timing unit 8 for determining a switching timing of the operation of the tachyarrhythmia prevention unit 6 by means of the control unit 7, a bradycardia treatment unit 9 for carrying out bradycardia treatment when bradycardia was detected by the heart contraction detection unit 2, a pacing pulse generation unit 10 for receiving a signal from the bradycardia treatment unit 9 and for generating a pacing pulse, a shock pulse generation unit 11 for applying a shock pulse to the heart 38, and a tachyarrhythmia termination unit 12 for supplying a signal for terminating the tachyarrhythmia to the pacing pulse generation unit 10 and the shock pulse generation unit 11.

The tachyarrhythmia prevention unit 6 is constituted by two means of a sympathetic nerve stimulation unit 13 for supplying a stimulation signal to the ganglion stellatum 44 and a parasympathetic nerve stimulation unit 14 for supplying a stimulation signal to the vagus nerve 41 and is operated by a tachyarrhythmia prevention program. Then, a sympathetic nerve stimulation unit 13 is constituted by a blocking stimulation unit 15 and a sub-threshold stimulation unit 16 and a parasympathetic nerve stimulation unit 14 is constituted by an activating stimulation unit 17. Here, the blocking stimulation unit 15 is a unit for preventing accentuation of sympathetic nerve tension which heightens a risk of a tachyarrhythmia occurrence from being transmitted from the brain to the heart 38 and the sub-threshold stimulation unit 16 is a unit for restructuring the heart 38 to an organ characteristic in which tachyarrhythmia is seldom caused by applying a weak stimulation of a level which does not increase a heart rate or a blood pressure to the ganglion stellatum 44 with respect to the heart 38 having an organ characteristic in which it is easy for tachyarrhythmia to occur particularly such as after a myocardial infarction and by stimulating a nerve figuration in a sinus node (not shown) or a ventricle muscle. Usually, the sub-threshold stimulation unit 16 is operated and the blocking stimulation unit 15 will be operated in order to block it in a case when accentuation of a sympathetic nerve tension such as a heart rate increase or the like is detected. On the other hand, an activating stimulation unit 17 of a parasympathetic nerve stimulation unit 14 stimulates the vagus nerve 41 by a detection of a sympathetic nerve tension accentuation such as a heart rate increase or the like and represses the sympathetic nerve tension antagonistically and at the same time, reduces a risk of tachyarrhythmia occurrence by lowering oxygen consumption of the heart 38 after lowering the heart rate.

The control unit 7 is constituted by a deactivation unit 18 configured to cancel the preventive treatment by the tachyarrhythmia prevention unit 6 based on receiving a signal from the tachyarrhythmia detection unit 3 or the tachyarrhythmia confirmation unit 4 and an activation unit 19 for carrying out the preventive treatment by the tachyarrhythmia prevention unit 6 according to a signal from the timing unit 8. Outputs of the deactivation unit 18 and the activation unit 19 are supplied to any one of the blocking stimulation unit 15, the sub-threshold stimulation unit 16 and the activating stimulation unit 17 in the tachyarrhythmia prevention unit 6.

Also, the timing unit 8 is constituted by a tachyarrhythmia preventing treatment change-over timer 20 for starting clocking by an output the tachyarrhythmia termination confirmation unit 5 which confirms termination of the tachyarrhythmia, a change-over time memory unit 21 for storing a period from the termination confirmation of the tachyarrhythmia until the tachyarrhythmia preventing treatment is started, and a comparator unit 22 for emanating an output with respect to the activation unit 19 of the control unit 7 when the clocking time of the tachyarrhythmia preventing treatment change-over timer 20 reaches the time period stored in the change-over time memory unit 21.

The bradycardia treatment unit 9 is a so-called a pacemaker and is constituted by a first bradycardia treatment unit 23 for carrying out a bradycardia treatment at a normal time, a second bradycardia treatment unit 24 for carrying out a bradycardia treatment after the tachyarrhythmia termination in which a stimulation parameter of a stimulation rate, a stimulation volatage or the like is different from that in the first bradycardia treatment unit 23, a bradycardia treatment change-over timer 25 for starting clocking by an output of the tachyarrhythmia termination confirmation unit 5, a bradycardia treatment change-over time memory unit 26 for storing time at which the bradycardia treatment after the tachyarrhythmia termination is changed-over from the second bradycardia treatment unit 24 to the first bradycardia treatment unit 23, a comparator unit 27 for emanating an output when the clocking time of the bradycardia treatment change-over timer 25 reaches the time stored in the bradycardia treatment change-over time memory unit 26, and a bradycardia treatment selection unit 28 for receiving the output from the comparator unit 27 and for changing-over from the second bradycardia treatment unit 24 to the first bradycardia treatment unit 23.

Also, the tachyarrhythmia termination unit 12 is constituted by an anti-tachyarrhythmia pacing unit 30 which is effective for a slow tachycardia, a cardioversion unit 31 for carrying out direct-current power distribution in synchronism with heart contraction detection with respect to a rapid tachycardia, a defibrillation unit 32 for carrying out direct-current power distribution with respect to fibrillation and an anti-tachyarrhythmia treatment output change-over unit 33 for selecting either one of the anti-tachyarrhythmia pacing unit 30, the cardioversion unit 31 and the defibrillation unit 32 according to the output of the tachyarrhythmia detecton unit 3.

Here, the anti-tachyarrhythmia pacing unit 30 is a unit for stimulating the heart 38 at a rate higher than the detected tachyarrhythmia rate with respect to a relatively slow tachycardia after a predetermined coupling time from a heart contraction detection and the stimulation of the heart 38 is carried out by being connected to the pacing pulse generation unit 10 through the anti-tachyarrhythmia treatment output change-over unit 33. Also, the cardioversion unit 31 is a unit for carrying out direct-current power distribution with respect to a relatively rapid tachycardia in synchronism with the heart contraction detection and the direct-current power distribution is carried out by being connected to the shock pulse generation unit 11 through the anti-tachyarrhythmia treatment output change-over unit 33. Then, the defibrillation unit 32 is a unit for carrying out direct-current power distribution with respect to fibrillation and at that time, if it is possible, the direct-current power distribution is to be carried out in synchronism with the heart contraction detection. This direct-current power distribution, similarly as the cardioversion, is carried out by being connected to the shock pulse generation unit 11 through the anti-tachyarrhythmia treatment output change-over unit 33. In this manner, the anti-tachyarrhythmia treatment output change-over unit 33 is change-over means for being responsive to the output of the tachyarrhythmia detection unit 3 and for carrying out a connection by changing-over the selected treatment to the pacing pulse generation unit 10 or the shock pulse generation unit 11.

Here, the pacing pulse generation unit 10 is a unit for generating a stimulation output for the bradycardia treatment and the anti-tachyarrhythmia pacing treatment and usually, a pulse having pulse width of 1msec and amplitude of 10V at the maximum is emanated. Since the stimulation threshold becomes high during the tachyarrhythmia, the stimulation output of the anti-tachyarrhythmia pacing treatment is set to be higher than that of a usual bradycardia treatment. Also, the shock pulse generation unit 11 is a unit to be used as a stimulation output of the cardioversion treatment and the defibrillation treatment and usually, energy of 0.1 to 5 joule for the cardioversion or energy of 30 joule at the maximum for the defibrillation is emanated as a monophasic or biphasic pulse having pulse width of 6msec. Usually, a biphasic pulse which is superior for efficiency of the defibrillation is selected. A voltage of approximately 700 volts at the maximum is applied for the defibrillation treatment.

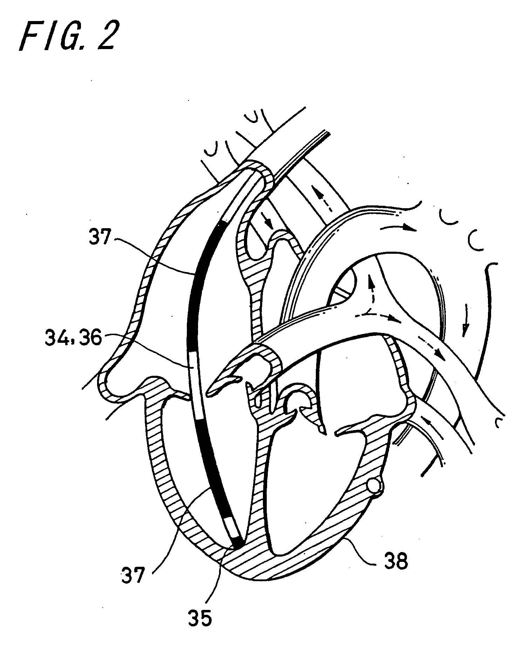

The heart contraction detection unit 2 and the pacing pulse generation unit 10 are connected to a heart stimulation/detection electrode 35 through a common heart electrode lead 34 and the shock pulse generation unit 11 is connected with an electrode 37 for supplying a shock pulse through a heart electrode lead 36. FIG. 2 shows an arrangement relation of the heart stimulation/detection electrode 35 and the electrode 37 for supplying a shock pulse in the heart. FIG. 2 is an example in which the heart stimulation/detection electrode 35 and the electrode 37 for supplying a shock pulse are arranged by means of a catheter electrode. The catheter electrode is introduced from the large vein to the right atrium of the heart 38, enters into the right ventricle passing through the auriculoventricular valve and the tip portion thereof is placed therein so as to be contacted with the lowermost portion of the right ventricle. This catheter electrode is covered by a sheath such as silicon, urethane or the like which has biocompatibility and the heart electrode lead 34 and the heart electrode lead 36 which are insulated electrically each other pass through the inside thereof. The heart stimulation/detection electrode 35 is arranged at the tip portion of the catheter electrode and connected with the heart electrode lead 34. Also, the electrodes 37 for supplying a shock pulse each of which has a large surface area are arranged at a portion on which the catheter electrode is positioned in the right ventricle and in the right atrium to the large vein and they are connected with the heart electrode lead 36. The electrode 37 for supplying a shock pulse is constituted as a coil shaped structure and it can be placed flexibly even in a heart.

In the example of this FIG. 2, owing to a fact that the heart detection electrode 35 arranged at the lowermost portion in the right ventricle observes the heart activity full-time, ventricular tachycardia or ventricular fibrillation is detected, and an anti-tachyarrhythmia pacing treatment is to be carried out with respect to a slow ventricular tachycardia through the heart stimulation electrode 35 arranged at the lowermost portion in the right ventricle and a cardioversion treatment or a defibrillation treatment is to be carried out with respect to a rapid ventricular tachycardia or a ventricular fibrillation by the shock pulse generation unit 11 between two apart electrodes of the electrodes 37 for supplying a shock pulse or between the electrode 37 for supplying a shock pulse and a case which is not shown of the heart treatment equipment 1.

Also, it may be constituted such that the supraventricular tachycardia or the atrial fibrillation is detected by arranging the heart stimulation/detection electrode 35 and the electrode 37 for supplying a shock pulse within the atrium and an anti-tachyarrhythmia pacing treatment is to be carried out with respect to a slow supraventricular tachycardia through the heart stimulation electrode 35 arranged in the right atrium and a cardioversion treatment or a defibrillation treatment is to be carried out with respect to a rapid supraventricular tachycardia or an atrial fibrillation by the shock pulse generation unit 11 between the electrode 37 for supplying a shock pulse and a case which is not shown of the heart treatment equipment 1.

In addition, the parasympathetic nerve stimulation unit 14 of the tachyarrhythmia prevention unit 6 is connected to a vagus nerve stimulation electrode 40 by way of a parasympathetic nerve electrode lead 39 and also, the sympathetic nerve stimulation unit 13 is connected to a sympathetic nerve stimulation electrode 43 through a sympathetic nerve electrode lead 42. The vagus nerve stimulation electrode 40 is fixed in a state that it is wound around the vagus nerve 41. A right central position of a neck portion region or an external carotid artery is preferable for a region on which the vagus nerve stimulation electrode 40 is wound around. Also, the sympathetic nerve stimulation electrode 43 is fixed by being wound around a body portion which is a thick portion of the ganglion stellatum 44 having a flat star shape. In addition, it is also possible to stimulate by winding it directly around the heart nerve 45 coming out of the ganglion stellatum 44.

Hereinafter, it will be explained with respect to the operation of the first embodiment of the heart treatment equipment according to the present invention shown in FIG. 1. First, a heart (ventricle) contraction is detected by the heart contraction detection unit 2. The heart contraction information detected by the heart contraction detection unit 2 is transmitted to the tachyarrhythmia detection unit 3, the tachyarrhythmia confirmation unit 4 and the tachyarrhythmia termination confirmation unit 5 and at the same time, it is supplied to the bradycardia treatment unit 9, the tachyarrhythmia prevention unit 6 and the tachyarrhythmia termination unit 12.

The tachyarrhythmia detection unit 3 observes the output of the heart contraction detection unit 2, detects a tachyarrhythmia and at the same time, makes a classification depending on the degree of the tachyarrhythmia, and an anti-tachyarrhythmia treatment corresponding to the situation of the classified tachyarrhythmia is to be selected. More specifically, the tachyarrhythmia which is heartbeats of a predetermined rate or more and is detected by the tachyarrhythmia detection unit 3 is further classified into a tachycardia and a fibrillation according to the degree of its rate. Then, further, the tachycardia is classified into a slow tachycardia and a rapid tachycardia, and there are applied an anti-tachyarrhythmia pacing treatment for the slow tachycardia, a cardioversion treatment for the rapid tachycardia and a defibrillation treatment for the fibrillation correspondingly. With respect to the correspondence making of the tachyarrhythmia detection/classify/treatment method, it is also possible to add criterion such as rate stability (whether or not the tachyarrhythmia rate is stable), suddenness (whether or not the tachyarrhythmia was caused suddenly), an electrocardiogram configuration (QRS width) or the like. When a corresponding anti-tachyarrhythmia treatment is selected according to the detection/classification of the tachyarrhythmia, a switching instruction to a selected anti-tachyarrhythmia treatment is transmitted from the tachyarrhythmia detection unit 3 with respect to the anti-tachyarrhythmia treatment output change-over unit 33 of the tachyarrhythmia termination unit 12. Then, in a case when the selected anti-tachyarrhythmia treatment is an anti-tachyarrhythmia pacing, an output is transmitted from the anti-tachyarrhythmia treatment output change-over unit 33 with respect to the pacing pulse generation unit 10 and at the same time, an output is also transmitted with respect to the tachyarrhythmia termination confirmation unit 5 and a tachyarrhythmia termination confirmation is carried out.

As described above, when a rapid tachycardia is detected by the tachyarrhythmia detection unit 3, a cardioversion treatment is to be applied and when a fibrillation is detected, a defibrillation treatment is to be applied and, in this case, a process is requested in which the output of the heart contraction detection unit 2 is observed and it is confirmed by the tachyarrhythmia confirmation unit 4 whether or not the detected tachyarrhythmia continues during a period until a predetermined amount of energy corresponding to the cardioversion treatment or the defibrillation treatment is charged in a capacitor which is not shown in the shock pulse generation unit 11. Generally, 1 to 2 seconds are required for the charging time by the cardioversion treatment and 7 to 9 seconds for the defibrillation treatment. Then, only in a case when it is confirmed that the tachyarrhythmia continues until a time point at which the charging to the capacitor in the shock pulse generation unit 11 is completed, an execution instruction of the cardioversion treatment or the defibrillation treatment is transmitted from the tachyarrhythmia confirmation unit 4 with respect to the anti-tachyarrhythmia treatment output change-over unit 33 of the tachyarrhythmia termination unit 12 and at the same time, an instruction is transmitted with respect to the deactivation unit 18 of the control unit 7 so as to deactivate the tachyarrhythmia prevention unit 6. On the other hand, when a spontaneous termination of the detected tachyarrhythmia is confirmed, an instruction for releasing the selected cardioversion treatment or defibrillation treatment is transmitted with respect to the anti-tachyarrhythmia treatment output change-over unit 33 of the tachyarrhythmia termination unit 12.

The tachyarrhythmia termination confirmation unit 5 receives a signal showing an execution of a selected anti-tachyarrhythmia treatment from the anti-tachyarrhythmia treatment output change-over unit 33 of the tachyarrhythmia termination unit 12, observes the output of the heart contraction detection unit 2 and when it is confirmed that the detected tachyarrhythmia terminated, the tachyarrhythmia preventing treatment change-over timer 20 of the timing unit 8 and the bradycardia treatment change-over timer 25 of the bradycardia treatment unit 9 are made to start thereby.

When receiving a instruction for deactivating tachyarrhythmia prevention from the tachyarrhythmia detection unit 3 or the tachyarrhythmia confirmation unit 4, the deactivation unit 18 of the control unit 7 emanates a signal to the tachyarrhythmia prevention unit 6, and the stimulation to the vagus nerve 41 by the activating stimulation unit 17 and the stimulation to the ganglion stellatum 44 by the blocking stimulation unit 15 and the sub-threshold stimulation unit 16 are made to stop thereby.

Also, the anti-tachyarrhythmia treatment output change-over unit 33 of the tachyarrhythmia termination unit 12 obtains an output from the tachyarrhythmia detection unit 3 or the tachyarrhythmia confirmation unit 4, selects any one of the anti-tachyarrhythmia pacing treatment, the cardioversion treatment and the defibrillation treatment in response to the tachyarrhythmia classification detected by the tachyarrhythmia detection unit 3 and in case of an anti-tachyarrhythmia pacing treatment, it supplies an output of the anti-tachyarrhythmia pacing unit 30 to the pacing pulse generation unit 10. Then, when the instruction from the tachyarrhythmia detection unit 3 is a cardioversion treatment or a defibrillation treatment, the anti-tachyarrhythmia treatment output change-over unit 33 selects an output of the cardioversion unit 31 or the defibrillation unit 32 and supplies it to the shock pulse generation unit 11.

In a case when the anti-tachyarrhythmia pacing treatment is selected, the anti-tachyarrhythmia treatment output change-over unit 33 connects the anti-tachyarrhythmia pacing unit 30 to the pacing pulse generation unit 10 and starts an anti-tachyarrhythmia pacing treatment at once. In a case when a cardioversion or a defibrillation treatment is selected, the cardioversion unit 31 or the defibrillation unit 32 is connected to the shock pulse generation unit 11, an amount of energy corresponding to each treatment is set in the shock pulse generation unit 11 and charging is started. When it is transmitted from the tachyarrhythmia confirmation unit 4 during the charging operation or after completion of the charging that the tachyarrhythmia termination is confirmed, a charging stop instruction and an instruction of internal discharge are carried out with respect to the shock pulse generation unit 11. Then, the connection between the cardioversion unit 31 or the defibrillation unit 32 and the shock pulse generation unit 11 is to be released. On the other hand, when it is confirmed in the tachyarrhythmia confirmation unit 4 that the tachyarrhythmia continues after the charging completion in the shock pulse generation unit 11, an instruction for carrying out direct-current power distribution to the heart 38 is transmitted with respect to the shock pulse generation unit 11.

Next, the bradycardia treatment unit 9 is a unit for carrying out a usual bradycardia treatment and a bradycardia treatment after a tachyarrhythmia termination confirmation differently and the first bradycardia treatment unit 23 is used for the usual bradycardia treatment. More specifically, the first bradycardia treatment unit 23 has a function of a usual pacemaker for carrying out a stimulation of the heart 38 by a predetermined rate in a case when the output of the heart contraction detection unit 2 goes under the predetermined rate. The stimulation of the heart 38 in this case is carried out by the pacing pulse generation unit 10 through the bradycardia treatment selection unit 28.

Also, the bradycardia treatment after the tachyarrhythmia termination confirmation is carried out by the second bradycardia treatment unit 24. In the second bradycardia treatment unit 24, the stimulation output thereof is set to be high as compared with that of a usual bradycardia treatment carried out in the first bradycardia treatment unit 23 by being preparing for an increase of a stimulation threshold of the heart 38 (necessary stimulation energy for beating the heart 38) after an anti-tachyarrhythmia treatment. Also, it is also possible to set the stimulation rate to be high for an early recovery of hemodynamics after an anti-tachyarrhythmia treatment. Further, it is constituted for preventing tachyarrhythmia recurrence by heart stimulation just after a tachyarrhythmia termination such that it can be set for the bradycardia treatment to be started at whichever time point after the tachyarrhythmia termination.

The change over of the first bradycardia treatment unit 23 and the second bradycardia treatment unit 24 is carried out by the bradycardia treatment selection unit 28. More specifically, when a signal from the tachyarrhythmia termination confirmation unit 5 is supplied to the bradycardia treatment change-over timer 25, the bradycardia treatment change-over timer 25 resets the clocking content until that moment and starts clocking. In synchronism with the start of this bradycardia treatment change-over timer 25, the bradycardia treatment selection unit 28 selects the second bradycardia treatment unit 24 in order to apply a necessary bradycardia treatment after a tachyarrhythmia termination confirmation. Then, when the clocking time of the bradycardia treatment change-over timer 25 reaches the time stored in the bradycardia treatment change-over time memory unit 26, the comparator unit 27 emanates an output and receiving this output, the bradycardia treatment selection unit 28 changes-over from the second bradycardia treatment unit 24 again to the first bradycardia treatment unit 23 which is a usual bradycardia treatment. Also, the output signal of the comparator unit 27 is also supplied to the bradycardia treatment change-over timer 25, it stops the clocking of the bradycardia treatment change-over timer 25 and at the same time resets the clocking content thereof.

Next, the operation of the timing unit 8 will be explained. First, when a tachyarrhythmia termination confirmation signal from the tachyarrhythmia termination confirmation unit 5 is supplied to the tachyarrhythmia preventing treatment change-over timer 20 of the timing unit 8, the tachyarrhythmia preventing treatment change-over timer 20 resets the clocking content until that moment and starts the clocking. Then, when the tachyarrhythmia preventing treatment change-over timer 20 makes clocking of a predetermined time after a tacyarrhythmia termiantion confirmation, that is, the time stored in the change-over time memory unit 21, the comparator unit 22 emanates an output to the activation unit 19 of the control unit 7, a tacyarrhythmia prevention treatment by the tachyarrhythmia prevention unit 6 is restarted. At that time, the output signal of the comparator unit 22 is also supplied to the tachyarrhythmia preventing treatment change-over timer 20, so that the clocking of the tachyarrhythmia preventing treatment change-over timer 20 is stopped and at the same time, its clocking content is reset.

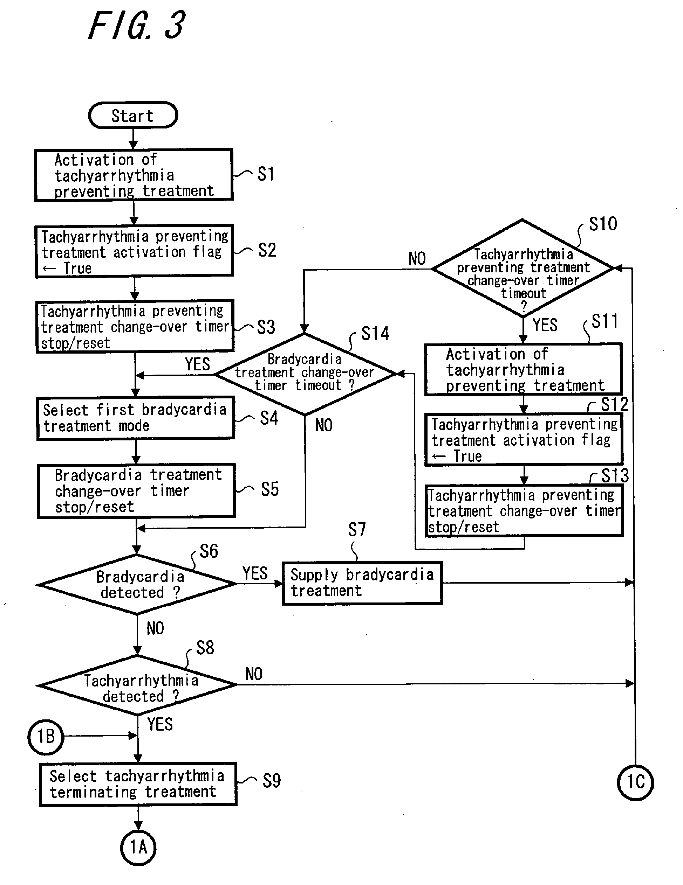

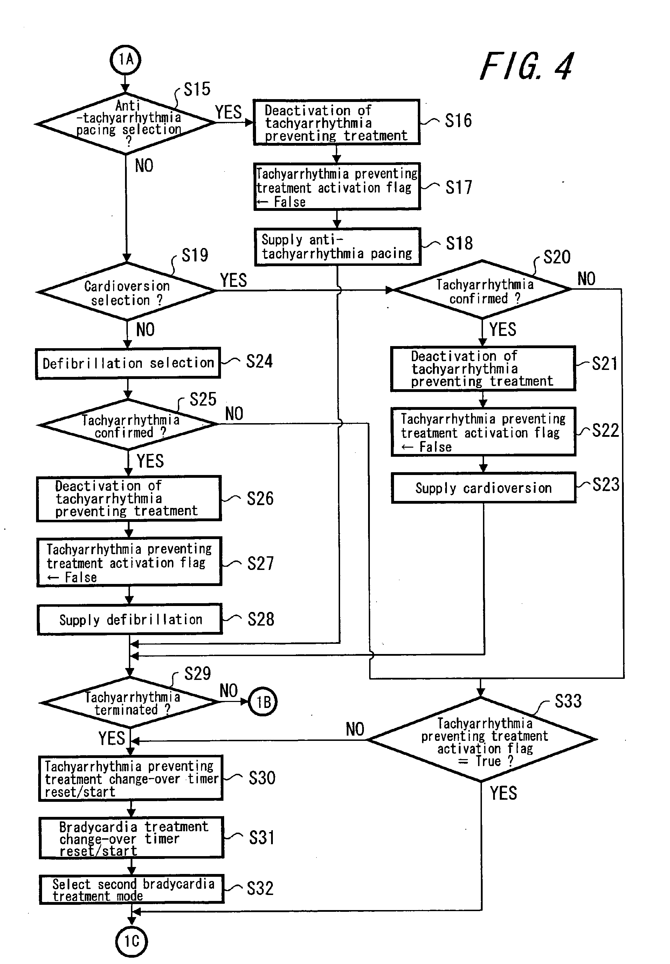

Hereinafter, the operation of the first exemplified embodiment according to the present invention will be explained in detail by using flow diagrams shown in FIG. 3 and FIG. 4.

First, as shown in FIG. 3, a tachyarrhythmia preventing treatment program of the tachyarrhythmia prevention unit 6 is activated by a signal from the activation unit 19 of the control unit 7 (step S1) and at the same time, "True" is written in an tachyarrhythmia preventing treatment activation flag, which is not shown, of the tachyarrhythmia prevention unit 6 (step S2). Next, in the timing unit 8, the tachyarrhythmia preventing treatment change-over timer 20 is stopped/reset (step S3), in the bradycardia treatment unit 9, the first bradycardia treatment mode of the first bradycardia treatment unit 23 is selected by the bradycardia treatment selection unit 28 (step S4) and at the same time, the bradycardia treatment change-over timer 25 is stopped/reset (step S5).

Next, in the heart contraction detection unit 2, it is judged whether or not a bradycardia was detected (step S6) and in a case when a bradycardia was detected, a bradycardia treatment is supplied by the pacing pulse generation unit 10 with respect to the heart 38 (step S7). In a case in judgment step S6 when a bradycardia was not detected, it is judged in the tachyarrhythmia detection unit 3 subsequently whether or not a tachyarrhythmia was detected (step S8) and in a case when a tachyarrhythmia was detected, a signal is transmitted to the tachyarrhythmia termination unit 12 and a tachyarrhythmia terminating treatment corresponding to the detected tachyarrhythmia is selected among the anti-tachyarrhythmia pacing, the cardioversion and the defibrillation (step S9). Thereafter, the flow proceeds to 1A of FIG. 4.

In a case when a bradycardia treatment was supplied in step S7 or when a tachyarrhythmia was not detected in judgment step S8, it is judged whether or not the tachyarrhythmia preventing treatment change-over timer 20 of the timing unit 8 is timeout (step S10). In a case when the tachyarrhythmia preventing treatment change-over timer 20 is timeout, that is, in a case when the tachyarrhythmia preventing treatment change-over timer 20 made clocking beyond the time stored in the change-over time memory unit 21, a signal is transmitted to the activation unit 19 of the control unit 7 so as to activate the tachyarrhythmia preventing treatment program (step S11) and at the same time, "True" is written in the tachyarrhythmia preventing treatment activation flag of the tachyarrhythmia prevention unit 6 (step S12), and subsequently, the tachyarrhythmia preventing treatment change-over timer 20 is stopped/reset (step S13).

Next, in a case when it is judged in judgment step S10 that the tachyarrhythmia preventing treatment change-over timer 20 is not timeout and after the tachyarrhythmia preventing treatment change-over timer 20 is stopped/reset in step S13, it is judged whether or not the bradycardia treatment change-over timer 25 in the bradycardia treatment unit 9 is timeout, that is, whether or not the bradycardia treatment change-over timer 25 made clocking beyond the time stored in the bradycardia treatment change-over time memory unit 26 (step S14).

Then, when it is judged that the bradycardia treatment change-over timer 25 is timeout, the flow returns to the selection of the first bradycardia treatment mode in step S4 and in a case when the bradycardia treatment change-over timer 25 is not timeout, a new bradycardia detection is waited for in judgment step S6.

Next, it will be explained with respect to the operation of the first exemplified embodiment according to the present invention after the tachyarrhythmia terminating treatment was selected in step S9 according to a flow diagram of FIG. 4.

First, it is judged whether or not an anti-tachyarrhythmia pacing treatment is selected for the anti-tachyarrhythmia treatment (tachyarrhythmia terminating treatment) in the tachyarrhythmia termination unit 12 (step S15). In a case when the anti-tachyarrhythmia pacing treatment is selected, the deactivation unit 18 of the control unit 7 is operated so as to deactivate the tachyarrhythmia preventing treatment of the tachyarrhythmia prevention unit 6 (step S16) and at the same time, "False" is written in the tachyarrhythmia preventing treatment activation flag of the tachyarrhythmia prevention unit 6 (step S17), and subsequently, the anti-tachyarrhythmia treatment output change-over unit 33 of the tachyarrhythmia termination unit 12 connects the anti-tachyarrhythmia pacing unit 30 and the pacing pulse generation unit 10 and an anti-tachyarrhythmia pacing is supplied with respect to the heart 38 (step S18).

In a case when an anti-tachyarrhythmia pacing is not selected in judgment step S15, it is judged next whether or not a cardioversion treatment is selected (step S19). In a case when the cardioversion treatment is selected, the anti-tachyarrhythmia treatment output change-over unit 33 of the tachyarrhythmia termination unit 12 connects the cardioversion unit 31 and the shock pulse generation unit 11 and charging of a predetermined amount of energy corresponding to the cardioversion treatment is carried out with respect to a capacitor of the shock pulse generation unit 11. In the tachyarrhythmia confirmation unit 4, it is confirmed for a period until the charging to this capacitor is completed whether or not the tachyarrhythmia is continuing (step S20). Then, in a case when the tachyarrhythmia is confirmed to be continuing, a signal is transmitted from the deactivation unit 18 of the control unit 7 to the tachyarrhythmia prevention unit 6 and the tachyarrhythmia preventing treatment program is stopped (step S21) and at the same time, "False" is written in the tachyarrhythmia preventing treatment activation flag of the tachyarrhythmia prevention unit 6 (step S22), and subsequently, a cardioversion treatment by the cardioversion unit 31 which was connected by the anti-tachyarrhythmia treatment output change-over unit 33 is supplied to the heart 38 through the shock pulse generation unit 11 (step S23).

When the anti-tachyarrhythmia pacing treatment is not selected in judgment step S15 and when the cardioversion treatment is not selected in judgment step S19, that is, when a defibrillation treatment is selected (step S24), the anti-tachyarrhythmia treatment output change-over unit 33 of the tachyarrhythmia termination unit 12 connects the defibrillation unit 32 and the shock pulse generation unit 11 and charging of a predetermined amount of energy corresponding to the defibrillation treatment is carried out with respect to a capacitor of the shock pulse generation unit 11. In the tachyarrhythmia confirmation unit 4, it is confirmed for a period until the charging to this capacitor is completed whether or not the tachyarrhythmia is continuing (step S25). Then, in a case when the tachyarrhythmia is confirmed to be continuing, the deactivation unit 18 of the control unit 7 is operated and the tachyarrhythmia preventing treatment of the tachyarrhythmia prevention unit 6 is stopped (step S26) and at the same time, "False" is written in the tachyarrhythmia preventing treatment activation flag of the tachyarrhythmia prevention unit 6 (step S27), and subsequently, a defibrillation treatment by the defibrillation unit 32 which was connected by the anti-tachyarrhythmia treatment output change-over unit 33 is supplied to the heart 38 through the shock pulse generation unit 11 (step S28).

Next, after the anti-tachyarrhythmia pacing treatment in step S18, the cardioversion treatment in step S23 or the defibrillation treatment in step S28 is supplied with respect to the heart 38, it is judged in the tachyarrhythmia termination confirmation unit 5 whether or not the tachyarrhythmia was terminated (step S29). Then, in a case when the tachyarrhythmia is confirmed to be terminated, the tachyarrhythmia preventing treatment change-over timer 20 of the timing unit 8 is reset/started (step S30), and subsequently, the bradycardia treatment change-over timer 25 of the bradycardia treatment unit 9 is reset/started (step S31) and at the same time, a second bradycardia treatment mode of the second bradycardia treatment unit 24 is selected by the bradycardia treatment selection unit 28 (step S32) and the flow returns to judgment step S10 in FIG. 3.

In a case when termination of the tachyarrhythmia is not confirmed in judgment step S29, the flow returns to step S9 in FIG. 3 and selection of the tachyarrhythmia terminating treatment is again carried out, but in the selection of this tachyarrhythmia termination treatment, augmentation of the applied energy, change of the tachyarrhythmia terminating treatment method (for example, treatment method change from cardioversion to defibrillation) or the like is carried out in a stepwise manner according to a predetermined treatment plan.

In a case when continuation of the tachyarrhythmia is not confirmed in judgment step S20 and judgment step S25, that is, in a case when the tachyarrhythmia terminated spontaneously amid the charging to the capacitor of the shock pulse generation unit 11, stop of the charging, internal discharge of the charging energy and releasing of the connection of the cardioversion unit 31 of the tachyarrhythmia termination unit 12 or the defibrillation unit 32 and the shock pulse generation unit 11 are carried out and at the same time, it is judged whether or not the tachyarrhythmia preventing treatment activation flag of the tachyarrhythmia prevention unit 6 is "True" (step S33). Then, in a case when the tachyarrhythmia preventing treatment activation flag is "True", that is, when the tachyarrhythmia preventing treatment of the tachyarrhythmia prevention unit 6 is activated, the flow returns to judgment step S10 in FIG. 3 and in a case when the tachyarrhythmia preventing treatment activation flag is not "True", that is, when the tachyarrhythmia preventing treatment of the tachyarrhythmia prevention unit 6 is not activated, it is assumed that the possibility that the heart is still in an unstable situation is high so that processes subsequent to step S30, which are sequences after the tachyarrhythmia termination is carried out through the tachyarrhythmia termination confirmation unit 5.

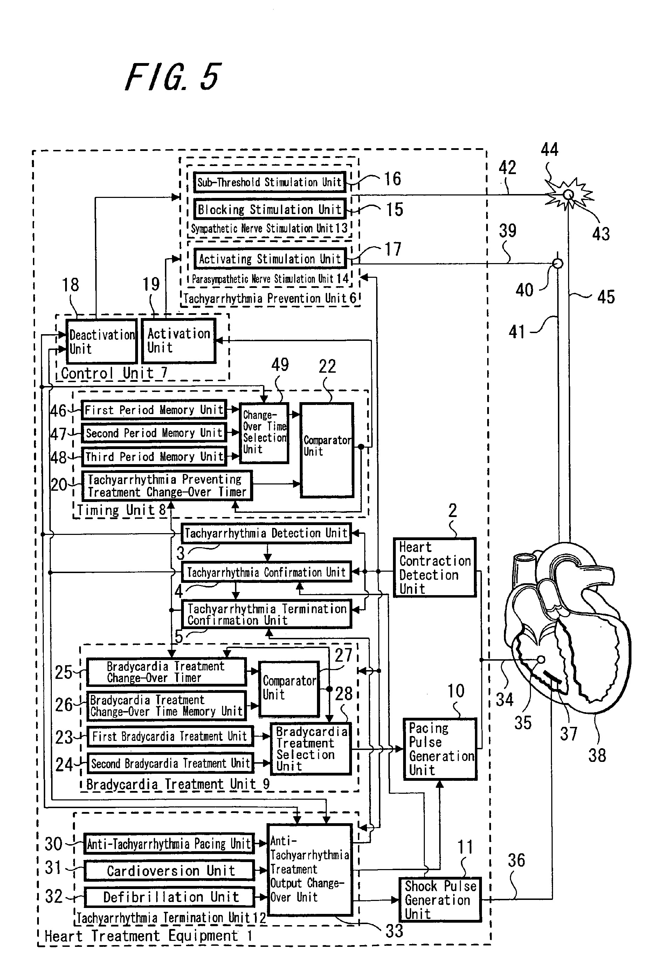

FIG. 5 is a block constitutional diagram showing a second exemplified embodiment of the heart treatment equipment according to the present invention. The portion which is different from that of the first exemplified embodiment shown in FIG. 1 lies in the constitution of the timing unit 8. Other constitutions are same as those of the block constitutional diagram in FIG. 1, so that the same reference numerals are put thereon and the explanation thereof will be omitted.

In the first exemplified embodiment of the present invention, the time period from the confirmation of the tachyarrhythmia termination to the restart of the tachyarrhythmia preventing treatment is stored in the change-over time memory unit 21 without relation to the tachyarrhythmia situation, but in the second exemplified embodiment of the present invention, the time period from the confirmation of aforesaid tachyarrhythmia termination to restart of the tachyarrhythmia preventing treatment is distinguished in three steps depending on the tachyarrhythmia situation. More specifically, the timing unit 8 in the second exemplified embodiment of the present invention is constituted by a tachyarrhythmia preventing treatment change-over timer 20 which starts clocking according to the output of the tachyarrthmia termination confirmation unit 5, a first period memory unit 46 which stores a time period from the tachyarrhythmia termination confirmation after the anti-tachyarrhythmia treatment by the anti-tachyarrhythmia pacing treatment until the tachyarrhythmia prevention unit 6 is activated, a second period memory unit 47 which stores a time period from the tachyarrhythmia termination confirmation after the anti-tachyarrhythmia treatment the cardioversion treatment until the tachyarrhythmia prevention unit 6 is activated, a third period memory unit 48 which stores a time period from the tachyarrhythmia termination confirmation after the anti-tachyarrhythmia treatment by the defibrillation treatment until the tachyarrhythmia prevention unit 6 is activated, a change-over time selection unit 49 for selecting any one of aforesaid first period memory unit 46 to the third period memory unit 48 depending on the tachyarrhythmia situation detected by the tachyarrhythmia detection unit 3 and for supplying it to the comparator unit 22, and a comparator unit 22 for comparing aforesaid selected changing-over time and the time which the tachyarrhythmia preventing treatment change-over timer 20 makes clocking and for supplying a signal to the activation unit 19 of the control unit 7 in a case when the time clocked in the tachyarrhythmia preventing treatment change-over timer 20 exceeds aforesaid selected stored time.

Usually, the stored value of the third period memory unit 48 which is a changing-over time with respect to the defibrillation treatment is set to be longer than the stored values of the second period memory unit 47 with respect to the cardioversion and of the first period memory unit 46 with respect to the anti-tachyarrhythmia pacing treatment and also, the stored value of the second period memory unit 47 which is a changing-over time with respect to the cardioversion treatment is set to be longer than the stored value of the first period memory unit 46 with respect to the anti-tachyarrhythmia pacing. This is because it can be considered such that the stronger energy is applied to the heart, the longer the time period in which the heart is unstable becomes.

According to the second exemplified embodiment of the present invention, when a tachyarrhythmia is detected in the tachyarrhythmia detection unit 3, any one is selected among the first period memory unit 46, the second period memory unit 47 and the third period memory unit 48 by the change-over time selection unit 49 depending on the tachyarrhythmia situation and the selected time is made to be a threshold of the comparator unit 22. Thereafter, when the tachyarrhythmia termination is confirmed in the tachyarrhythmia termination confirmation unit 5, the tachyarrhythmia preventing treatment change-over timer 20 starts clocking and when it reaches aforesaid selected threshold, the comparator unit 22 emanates an output to the activation unit 19 of the control unit 7 so as to restart the tachyarrhythmia preventing treatment by the tachyarrhythmia prevention unit 6.

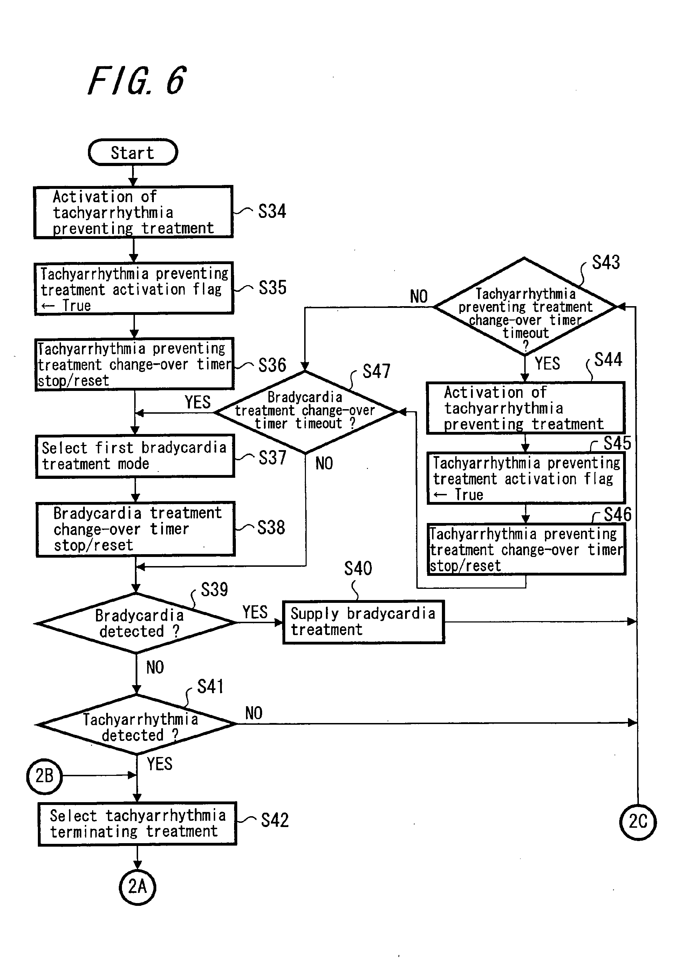

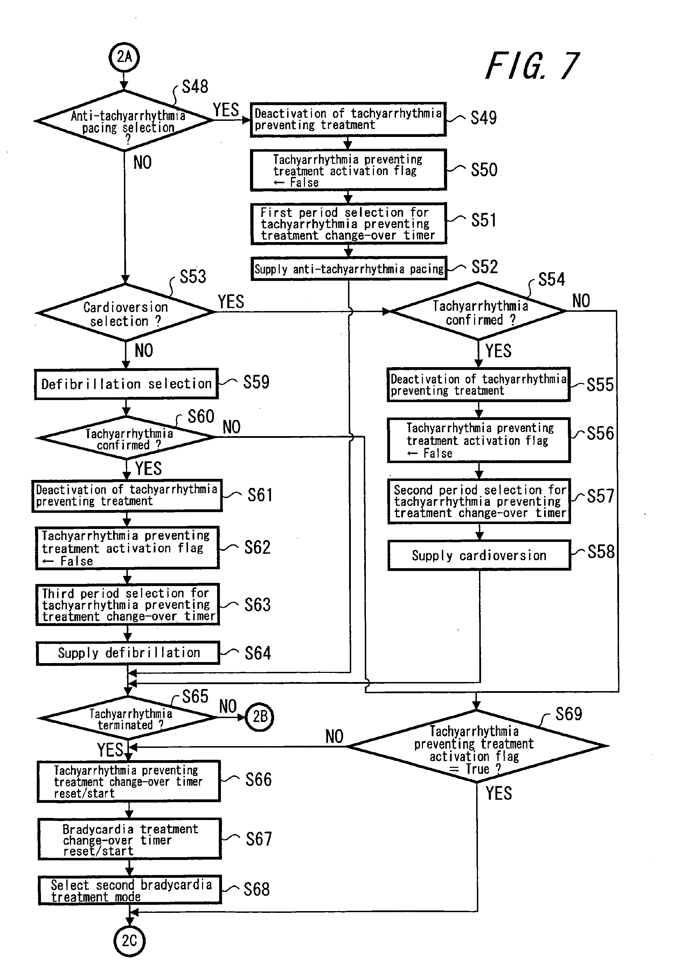

Hereinafter, it will be explained with respect to the operation of the second exemplified embodiment of the present invention in detail according to FIG. 6 and FIG. 7. The flow diagram of FIG. 6 is same as the flow diagram of FIG. 3, but reference numerals of respective steps therein are made to be different from those of the first exemplified embodiment, so that it will be explained also by including the repetitive portions thereof.

First, the tachyarrhythmia preventing treatment of the tachyarrhythmia prevention unit 6 is activated by the signal from the activation unit 19 of the control unit 7 (step S34) and at the same time, "True" is written in the tachyarrhythmia preventing treatment activation flag, which is not shown, of the tachyarrhythmia prevention unit 6 (step S35). Next, in the timing unit 8, the tachyarrhythmia preventing treatment change-over timer 20 is stopped/reset (step S36) and in the bradycardia treatment unit 9, the first bradycardia treatment mode of the first bradycardia treatment unit 23 is selected by the bradycardia treatment selection unit 28 (step S37) and at the same time, the bradycardia treatment change-over timer 25 is stopped/reset (step S38).

Next, it is judged whether or not a bradycardia was detected in the heart contraction detection unit 2 (step S39) and in a case when a bradycardia was detected, a bradycardia treatment is supplied by the pacing pulse generation unit 10 with respect to the heart 38 (step S40). In a case when a bradycardia was not detected in judgment step S39, it is judged subsequently whether or not a tachyarrhythmia was detected in the tachyarrhythmia detection unit 3 (step S41) and in a case when a tachyarrhythmia was detected, a signal is transmitted to the tachyarrhythmia termination unit 12 and a tachyarrhythmia terminating treatment corresponding to the detected tachyarrhythmia is selected among the anti-tachyarrhythmia pacing, the cardioversion and the defibrillation (step S42). Thereafter, the flow proceeds to 2A of FIG. 7.

In a case when a bradycardia treatment is supplied in step S40 or a tachyarrhythmia was not detected in judgment step S41, it is judged whether or not the tachyarrhythmia preventing treatment change-over timer 20 of the timing unit 8 is timeout (step S43). In a case when the tachyarrhythmia preventing treatment change-over timer 20 is timeout, that is, in a case when the tachyarrhythmia preventing treatment change-over timer 20 made clocking beyond the time stored in any one of the first period memory unit 46, the second period memory unit 47 or the third period memory unit 48 which is selected by the change-over time selection unit 49, a signal is transmitted to the activation unit 19 of the control unit 7 so as to activate the tachyarrhythmia preventing treatment program (step S44) and at the same time, "True" is written in the tachyarrhythmia preventing treatment activation flag of the tachyarrhythmia prevention unit 6 (step S45), and subsequently, the tachyarrhythmia preventing treatment change-over timer 20 is stopped/reset (step S46).

Next, in a case when it is judged in judgment step S43 that the tachyarrhythmia preventing treatment change-over timer 20 is not timeout and after the tachyarrhythmia preventing treatment change-over timer 20 was stopped/reset in step S46, it is judged whether or not the bradycardia treatment change-over timer 25 in the bradycardia treatment unit 9 is timeout, that is, whether or not the bradycardia treatment change-over timer 25 made clocking beyond the time stored in the bradycardia treatment change-over time memory unit 26 (step S47).

Then, when it is judged that the bradycardia treatment change-over timer 25 is timeout, the flow returns to the selection of the first bradycardia treatment mode in step S37 and in a case when the bradycardia treatment change-over timer 25 is not timeout, a new bradycardia detection is waited for in judgment step S39.

Next, it will be explained with respect to the operation of the second exemplified embodiment of the present invention after the selection of the tachyarrhythmia terminating treatment was carried out in step S42 according to a flow diagram of FIG. 7.

First, it is judged whether or not an anti-tachyarrhythmia pacing treatment is selected as the anti-tachyarrhythmia treatment (tachyarrhythmia terminating treatment) in the tachyarrhythmia termination unit 12 (step S48). In a case when the anti-tachyarrhythmia pacing treatment is selected, the deactivation unit 18 of the control unit 7 is operated so as to deavtivate the tachyarrhythmia preventing treatment program of the tachyarrhythmia prevention unit 6 (step S49) and at the same time, "False" is written in the tachyarrhythmia preventing treatment activation flag of the tachyarrhythmia prevention unit 6 (step S50), and subsequently, the change-over time selection unit 49 of the timing unit 8 selects the time stored in the first period memory unit 46 as a threshold time of the tachyarrhythmia preventing treatment change-over timer 20 (step S51) and thereafter, the anti-tachyarrhythmia treatment output change-over unit 33 of the tachyarrhythmia termination unit 12 connects the anti-tachyarrhythmia pacing unit 30 and the pacing pulse generation unit 10 and supplies anti-tachyarrhythmia pacing with respect to the heart 38 (step S52).

In a case when the anti-tachyarrhythmia pacing is not selected in judgment step S48, it is judged next whether or not a cardioversion treatment is selected (step S53). In a case when the cardioversion treatment is selected, the anti-tachyarrhythmia treatment output change-over unit 33 of the tachyarrhythmia termination unit 12 connects the cardioversion unit 31 and the shock pulse generation unit 11 and charging of a predetermined amount of energy corresponding to the cardioversion treatment is carried out with respect to the capacitor of the shock pulse generation unit 11. It is confirmed during the period until the charging to the capacitor is completed in the tachyarrhythmia confirmation unit 4 whether or not the tachyarrhythmia is continuing (step S54). Then, in a case when it was confirmed that the tachyarrhythmia is continuing, a signal is transmitted from the deactivation unit 18 of the control unit 7 to the tachyarrhythmia prevention unit 6 so as to deactivate the tachyarrhythmia preventing treatment program (step S55) and at the same time, "False" is written in the tachyarrhythmia preventing treatment activation flag of the tachyarrhythmia prevention unit 6 (step S56), and subsequently, the change-over time selection unit 49 of the timing unit 8 selects the time stored in the second period memory unit 47 as the threshold time of the tachyarrhythmia preventing treatment change-over timer 20 (step S57) and thereafter, a cardioversion treatment by the cardioversion unit 31 is supplied to the heart 38 through the shock pulse generation unit 11 (step S58).

When the anti-tachyarrhythmia pacing treatment is not selected in judgment step s48 and when the cardioversion treatment is not selected in judgment step S53 either, that is, when the defibrillation treatment is selected (step S59), the anti-tachyarrhythmia treatment output change-over unit 33 of the tachyarrhythmia termination unit 12 connects the defibrillation unit 32 and the shock pulse generation unit 11 and charging of a predetermined amount of energy corresponding to the defibrillation treatment is carried out with respect to the capacitor of the shock pulse generation unit 11. It is confirmed whether or not the tachyarrhythmia is continuing in the tachyarrhythmia confirmation unit 4 during the period until the charging to the capacitor is completed (step S60). Then, in a case when it is confirmed that the tachyarrhythmia is continuing, the deactivation unit 18 of the control unit 7 is operated so as to deactivate the tachyarrhythmia preventing treatment program of the tachyarrhythmia prevention unit 6 (step S61) and at the same time, "False" is written in the tachyarrhythmia preventing treatment activation flag of the tachyarrhythmia prevention unit 6 (step S62), and subsequently, the change-over time selection unit 49 of the timing unit 8 selects the time stored in the third period memory unit 48 as the threshold time of the tachyarrhythmia preventing treatment change-over timer 20 (step S63) and thereafter, a defibrillation treatment by the defibrillation unit 32 is supplied to the heart 38 through the shock pulse generation unit 11 (step S64).

Next, after the anti-tachyarrhythmia pacing treatment of step S52, the cardioversion treatment of step S58 or the defibrillation treatment of step S64 is supplied with respect to the heart 38, it is judged in the tachyarrhythmia termination confirmation unit 5 whether or not the tachyarrhythmia is terminated (step S65). Then, in a case when it is confirmed that the tachyarrhythmia terminated, the tachyarrhythmia preventing treatment change-over timer 20 of the timing unit 8 is reset/started (step S66) and subsequently, the bradycardia treatment change-over timer 25 of the bradycardia treatment unit 9 is reset/started(step S67) and at the same time, the second bradycardia treatment mode of the second bradycardia treatment unit 24 is selected by the bradycardia treatment selection unit 28 (step S68) and the flow returns to judgment step S43 of FIG. 6.

In a case when the tachyarrhythmia termination is not confirmed in judgment step S65, the flow returns to step S42 of FIG. 6 and selection of the tachyarrhythmia terminating treatment is again carried out, but in this selection of the tachyarrhythmia terminating treatment, augmentation of the applied energy, change of the tachyarrhythmia terminating treatment method or the like is carried out in a stepwise manner according to a predetermined treatment plan.

In a case when continuation of the tachyarrhythmia is not confirmed in judgment step S54 and judgment step S60, that is, in a case when the tachyarrhythmia terminated spontaneously amid the charging to the capacitor of the shock pulse generation unit 11, releasing of charging stop, internal discharge of the charging energy and connection between the cardioversion unit 31 of the tachyarrhythmia termination unit 12 or the defibrillation unit 32 and the shock pulse generation unit 11 are carried out and at the same time, it is judged whether or not the tachyarrhythmia preventing treatment activation flag of the tachyarrhythmia prevention unit 6 is "True" (step S69). Then, in a case when the tachyarrhythmia preventing treatment activation flag is "True", that is, when the tachyarrhythmia preventing treatment of the tachyarrhythmia prevention unit 6 is activated, the flow returns to judgment step S43 of FIG. 6. On the other hand, in a case when the tachyarrhythmia preventing treatment activation flag is not "True", that is, when the tachyarrhythmia preventing treatment of the tachyarrhythmia prevention unit 6 is not activated, it is assumed that the possibility that the heart is still in an unstable situation is high so that processes subsequent to step S66, which are sequences after the tachyarrhythmia termination are carried out through the tachyarrhythmia termination confirmation unit 5. At that time, in the change-over time selection unit 49 of the timing unit 8, selection of any one of the first period memory unit 46, the second period memory unit 47 and the third period memory unit 48 corresponding to the tachyarrhythmia terminating treatment carried out just before is maintained and activation of the tachyarrhythmia preventing treatment of the tachyarrhythmia prevention unit 6 is controlled according to the time stored in this selected memory unit.

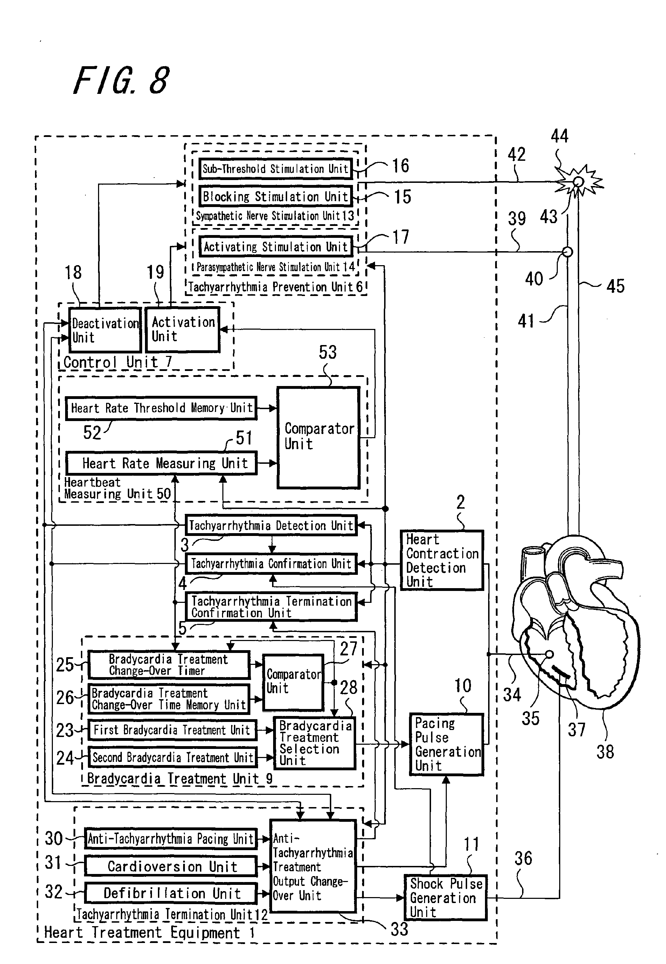

FIG. 8 is a block constitutional diagram showing a third exemplified embodiment of the heart treatment equipment according to the present invention. Portions which are different from those of the first exemplified embodiment of the present invention shown in FIG. 1 and those of the second exemplified embodiment shown in FIG. 5 lie in that a heartbeat measuring unit 50 is provided instead of the timing unit 8. Other constitutional portions are same as those of the block constitutional diagrams of FIG. 1 and FIG. 5, so that the same reference numerals are put thereon and the explanation thereof will be omitted.

More specifically, the heartbeat measuring unit 50 of the third exemplified embodiment according to the present invention is constituted by a heart rate measuring unit 51 supplied with an output of the heart contraction detection unit 2 and an output of the tachyarrhythmia termination confirmation unit 5, a heart rate threshold memory unit 52 for storing a predetermined heart rate threshold and a comparator unit 53 for emanating an output to the activation unit 19 of the control unit 7 when the measured value of the heart rate measuring unit 51 reaches the heart rate threshold stored in the heart rate threshold memory unit 52.

Then, the heart rate measuring unit 51 measures the heart rate detected by the heart contraction detection unit 2 after the tachyarrhythmia termination confirmation of the tachyarrhythmia termination confirmation unit 5 and when the measured numeric value reaches the heart rate threshold stored in the heart rate threshold memory unit 52, the comparator unit 53 transmits an output to the activation unit 19 and depending thereon, the tachyarrhythmia prevention unit 6 is activated and the tachyarrhythmia preventing treatment is carried out.

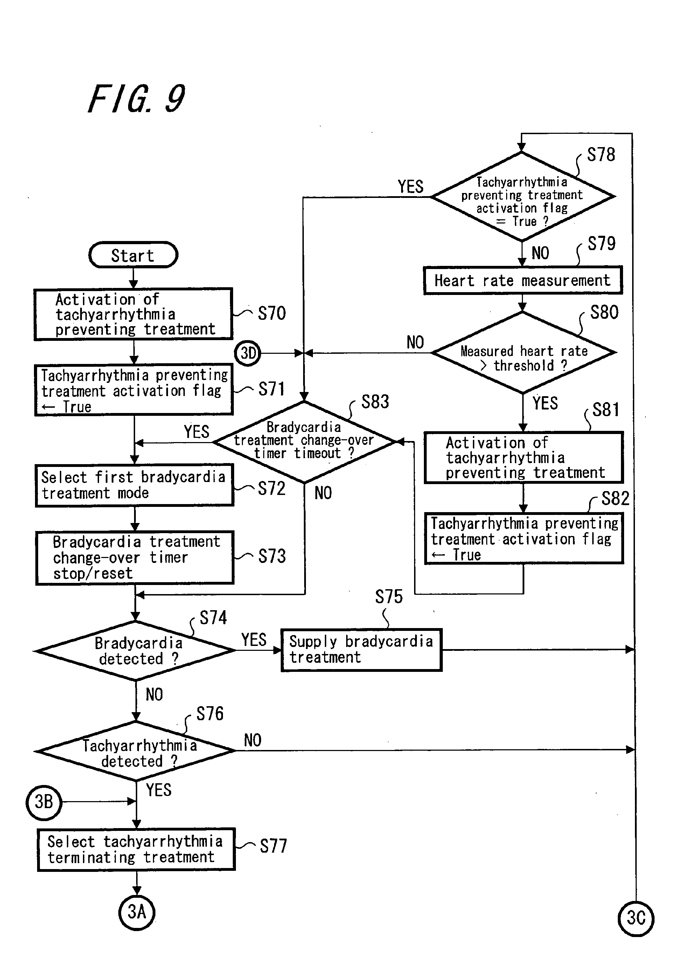

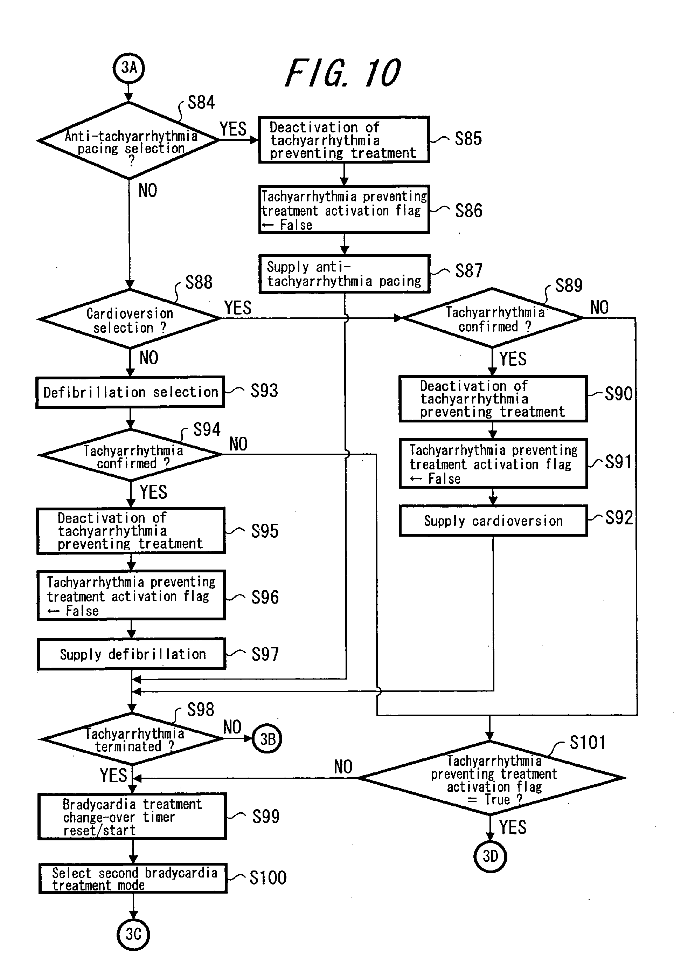

Hereinafter, it will be explained in detail with respect to the operation of the third exemplified embodiment of the present invention according to FIG. 9 and FIG. 10.

First, the tachyarrhythmia preventing treatment program of the tachyarrhythmia prevention unit 6 is actiated by the signal from the activation unit 19 of the control unit 7 (step S70) and at the same time, "True" is written in the tachyarrhythmia preventing treatment activation flag, which is not shown, of the tachyarrhythmia prevention unit 6 (step S71). Then, in the bradycardia treatment unit 9, the first bradycardia treatment mode of the first bradycardia treatment unit 23 is selected by the bradycardia treatment selection unit 28 (step S72) and at the same time, the bradycardia treatment change-over timer 25 is stopped/reset (step S73).

Next, it is judged in the heart contraction detection unit 2 whether or not a bradycardia was detected (step S74) and in a case when a bradycardia was detected, a bradycardia treatment is supplied by the pacing pulse generation unit 10 with respect to the heart 38 (step S75). In a case when a bradycardia was not detected in judgment step S74, it is judged subsequently whether or not a tachyarrhythmia was detected in the tachyarrhythmia detection unit 3 (step S76) and in a case when a tachyarrhythmia was detected, a signal is transmitted to the tachyarrhythmia termination unit 12, a tachyarrhythmia terminating treatment corresponding to the detected tachyarrhythmia is selected among the anti-tachyarrhythmia pacing, the cardioversion and the defibrillation (step S77), and the flow proceeds to 3A of FIG. 10.

In a case when a bradycardia treatment was supplied in step S75 or a tachyarrhythmia was not detected in judgment step S76, it is judged whether or not the tachyarrhythmia preventing treatment activation flag in the tachyarrhythmia prevention unit 6 is "True" (step S78). Then, in a case when the tachyarrhythmia preventing treatment activation flag is not "True", that is, when the tachyarrhythmia prevention treatment of the tachyarrhythmia prevention unit 6 is not activated, a heart rate measurement is carried out by the heart rate measuring unit 51 of the heartbeat measuring unit 50 (step S79) and it is judged whether or not this measured heart rate is bigger than the heart rate threshold stored in the heart rate threshold memory unit 52 (step S80).

Then, when the measured heart rate is bigger than the heart rate threshold, a control signal is transmitted to the activation unit 19 of the control unit 7 and the tachyarrhythmia preventing treatment of the tachyarrhythmia prevention unit 6 is carried out (step S81) and at the same time, the tachyarrhythmia preventing treatment activation flag of the tachyarrhythmia prevention unit 6 is change to "True" (step S82).

Next, in a case when the tachyarrhythmia preventing treatment activation flag was "True" in judgment step S78, in a case when it was judged in judgment step S80 that the measured heart rate is not bigger than the heart rate threshold and in a case when tachyarrhythmia preventing treatment was activated in step S81 and the tachyarrhythmia preventing treatment activation flag was changed to "True" in step S82, it is judged whether or not the bradycardia treatment change-over timer 25 in the bradycardia treatment unit 9 is timeout, that is, whether or not the bradycardia treatment change-over timer 25 made clocking beyond the time stored in the bradycardia treatment change-over time memory unit 26 (step S83).

Then, when it is judged that the bradycardia treatment change-over timer 25 is timeout, the flow returns to the selection of the first bradycardia treatment mode in step S72 and in a case when the bradycardia treatment change-over timer 25 is not timeout, a new bradycardia detection is waited for in judgment step S74.

Next, it will be explained with respect to the operation of the third exemplified embodiment of the present invention after the selection of the tachyarrhythmia terminating treatment was carried out in the step S77 (after 3A) according to the flow diagram of FIG. 10.

First, it is judged whether or not an anti-tachyarrhythmia pacing treatment is selected as an anti-tachyarrhythmia treatment (tachyarrhythmia terminating treatment) in the tachyarrhythmia termination unit 12 (step S84). In a case when an anti-tachyarrhythmia pacing treatment is selected, the deactivation unit 18 of the control unit 7 is operated so as to deactivate the tachyarrhythmia preventing treatment program of the tachyarrhythmia prevention unit 6 (step S85) and at the same time, "False" is written in the tachyarrhythmia preventing treatment activation flag of the tachyarrhythmia prevention unit 6 (step S86) and subsequently, the anti-tachyarrhythmia treatment output change-over unit 33 of the tachyarrhythmia termination unit 12 connects the anti-tachyarrhythmia pacing unit 30 and the pacing pulse generation unit 10 and an anti-tachyarrhythmia pacing is supplied with respect to the heart 38 (step S87).

In a case when the anti-tachyarrhythmia pacing is not selected in judgment step S84, it is judged next whether or not a cardioversion treatment is selected (step S88). In a case when the cardioversion treatment is selected, the anti-tachyarrhythmia treatment output change-over unit 33 of the tachyarrhythmia termination unit 12 connects the cardioversion unit 31 and the shock pulse generation unit 11 and charging of a predetermined amount of energy corresponding to the cardioversion treatment is carried out with respect to the capacitor of the shock pulse generation unit 11. It is confirmed whether or not the tachyarrhythmia is continuing in the tachyarrhythmia confirmation unit 4 during the period until the charging to the capacitor is completed (step S89). Then, in a case when it was confirmed that the tachyarrhythmia is continuing, a signal is transmitted from the deactivation unit 18 of the control unit 7 to the tachyarrhythmia prevention unit 6 so as to deactivate the tachyarrhythmia preventing treatment program (step S90) and at the same time, "False" is written in the tachyarrhythmia preventing treatment activation flag of the tachyarrhythmia prevention unit 6 (step S91) and subsequently, a cardioversion treatment by the cardioversion unit 31 which was connected by the anti-tachyarrhythmia treatment output change-over unit 33 is supplied to the heart 38 through the shock pulse generation unit 11 (step S92).

When the anti-tachyarrhythmia pacing treatment is not selected in judgment step S84 and when the cardioversion treatment is not selected in judgment step S88 either, that is, when the defibrillation treatment is selected (step S93), the anti-tachyarrhythmia treatment output change-over unit 33 of the tachyarrhythmia termination unit 12 connects the defibrillation unit 32 and the shock pulse generation unit 11 and charging of a predetermined amount of energy corresponding to the defibrillation treatment is carried out with respect to the capacitor of the shock pulse generation unit 11. It is confirmed whether or not the tachyarrhythmia is continuing in the tachyarrhythmia confirmation unit 4 during the period until the charging to the capacitor is completed (step S94). Then, in a case when it was confirmed that the tachyarrhythmia is continuing, the tachyarrhythmia preventing treatment of the tachyarrhythmia prevention unit 6 is canceled by operating the deactiavtion unit 18 of the control unit 7 (step S95) and at the same time, "False" is written in the tachyarrhythmia preventing treatment activation flag of the tachyarrhythmia prevention unit 6 (step S96) and subsequently, a defibrillation treatment by the defibrillation unit 32 which was connected by the anti-tachyarrhythmia treatment output change-over unit 33 is supplied to the heart 38 through the shock pulse generation unit 11 (step S97).

Next, after the anti-tachyarrhythmia pacing treatment of step S87, the cardioversion treatment of step S92 or the defibrillation treatment of step S97 is supplied with respect to the heart 38, it is judged in the tachyarrhythmia termination confirmation unit 5 whether or not the tachyarrhythmia is terminated (step S98). Then, in a case when it is confirmed that the tachyarrhythmia terminated, the bradycardia treatment change-over timer 25 of the bradycardia treatment unit 9 is reset/started (step S99) and at the same time, the second bradycardia treatment mode of the second bradycardia treatment unit 24 is selected by the bradycardia treatment selection unit 28 (step S100) and the flow returns to judgment step S78 of FIG. 9.

In a case when the tachyarrhythmia termination is not confirmed in judgment step S98, the flow returns to step S77 of FIG. 9 and selection of the tachyarrhythmia terminating treatment is again carried out, but in this selection of the tachyarrhythmia terminating treatment, augmentation of the applied energy, change of the tachyarrhythmia terminating treatment method or the like is carried out in a stepwise manner according to a predetermined treatment plan.

In a case when continuation of the tachyarrhythmia is not confirmed in judgment step S89 and judgment step S94, that is, in a case when the tachyarrhythmia terminated spontaneously amid the charging to the capacitor of the shock pulse generation unit 11, releasing of charging stop, internal discharge of the charging energy and connection between the cardioversion unit 31 of the tachyarrhythmia termination unit 12 or the defibrillation unit 32 and the shock pulse generation unit 11 are carried out and at the same time, it is judged whether or not the tachyarrhythmia preventing treatment activation flag of the tachyarrhythmia prevention unit 6 is "True" (step S101). Then, in a case when the tachyarrhythmia preventing treatment activation flag is "True", that is, when the tachyarrhythmia preventing treatment of the tachyarrhythmia prevention unit 6 is activated, the flow returns to judgment step S83 of FIG. 9 and in a case when the tachyarrhythmia preventing treatment activation flag is not "True", that is, when the tachyarrhythmia preventing treatment program of the tachyarrhythmia prevention unit 6 is not activated, it is assumed that the possibility that the heart is still in an unstable situation is high so that processes subsequent to step S99, which are sequences after the tachyarrhythmia termination are carried out through the tachyarrhythmia termination confirmation unit 5.

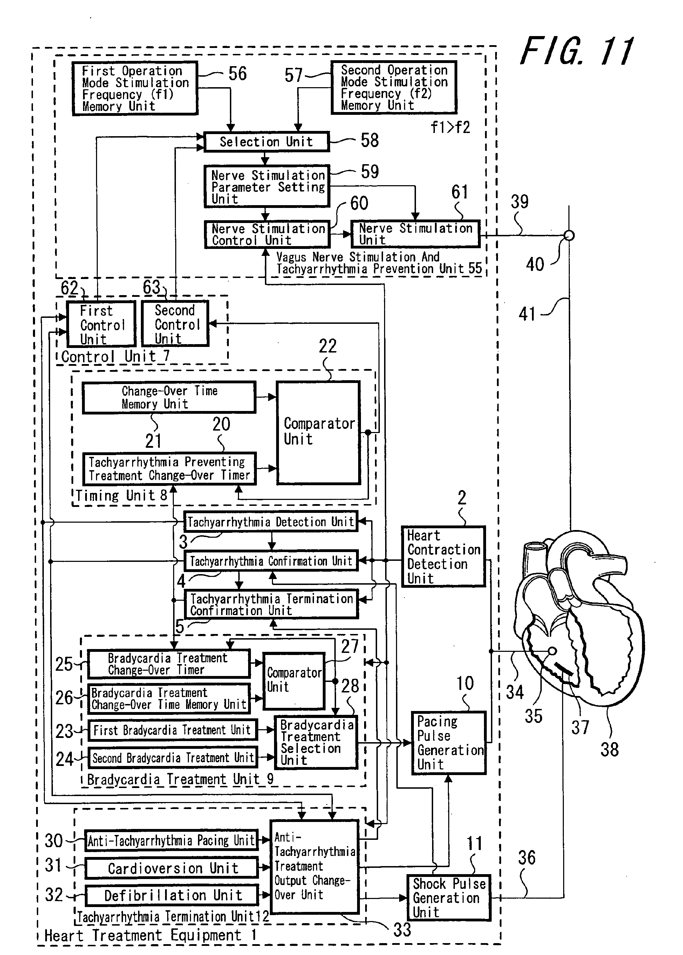

FIG. 11 is a block constitutional diagram showing a fourth exemplified embodiment of the heart treatment equipment according to the present invention. Portions which are different from those of the first exemplified embodiment of the present invention shown in FIG. 1 lie in the constitutions of the tachyarrhythmia prevention unit 6 and the control unit 7. More specifically, the tachyarrhythmia prevention unit 6 in this example is constituted for carrying out only vagus nerve stimulation and there is not provided with means for stimulating a sympathetic nerve such as a ganglion stellatum block or the like. Other constitutions are same as those of the block constitutional diagrams of FIG. 1, so that the same reference numerals are put thereon and the explanation thereof will be omitted.

As shown in FIG. 11, in the fourth exemplified embodiment of the present invention, a vagus nerve stimulation and tachyarrhythmia prevention unit 55 is used instead of the tachyarrhythmia prevention unit 6 of the first exemplified embodiment. It is possible for the vagus nerve stimulation and tachyarrhythmia prevention unit 55 to generate a plurality of stimulations having different stimulation frequencies with respect to the vagus nerve 41. More specifically, the vagus nerve stimulation and tachyarrhythmia prevention unit 55 of this example is constituted by a first operation mode stimulation frequency memory unit 56 for storing stimulation frequency f1 of the first operation mode, a second operation mode stimulation frequency memory unit 57 for storing stimulation frequency f2 of a second operation mode the frequency of which is lower than the stimulation frequency f1 of the first operation mode, a selection unit 58 for selecting either one of the stimulation frequency f1 of the first mode and the stimulation frequency f2 of the second mode, a nerve stimulation parameter setting unit 59 for setting the stimulation frequency selected by the selection unit 58 to a nerve stimulation signal parameter, a nerve stimulation control unit 60 for receiving an output of the heart contraction detection unit 2 and for controlling the stimulation of the vagus nerve 41, and a nerve stimulation unit 61 for being controlled by the nerve stimulation control unit 60 and for generating a nerve stimulation signal using the parameter which is set by the nerve stimulation parameter setting unit 59 with respect to the vagus nerve 41.

Also, the control unit 7 is constituted by a first control unit 62 for controlling the selection unit 58 of the vagus nerve stimulation and tachyarrhythmia prevention unit 55 in response to outputs of the tachyarrhythmia detection unit 3 and the tachyarrhythmia confirmation unit 4 and a second control unit 63 for receiving an output from the comparator unit 22 of the timing unit 8 and for supplying a signal to the selection unit 58 of the vagus nerve stimulation and tachyarrhythmia prevention unit 55.

It is programmed according to the fourth exemplified embodiment of the present invention shown in this FIG. 11 such that the selection unit 58 of the vagus nerve stimulation and tachyarrhythmia prevention unit 55 will select the second operation mode stimulation frequency f2 by the first control unit 62 of the control unit 7 on an occasion of the tachyarrhythmia detection by the tachyarrhythmia detection unit 3 or the tachyarrhythmia confirmation unit 4 and the stimulation of the vagus nerve 41 will be carried out by a relatively weak energy. However, when the tachyarrhythmia termination is confirmed in the tachyarrhythmia termination confirmation unit 5 and the time stored in the change-over time memory unit 21 elapses after the tachyarrhythmia preventing treatment change-over timer 20 starts clocking, an output is emanated from the comparator unit 22 to the second control unit 63 of the control unit 7 and the selection unit 58 of the vagus nerve stimulation and tachyarrhythmia prevention unit 55 selects the stimulation frequency f1 of the first operation mode. Then, it is constituted in this case such that the stimulation of the vagus nerve 41 is to be carried out by a relatively strong energy.

It should be noted in this example that a constitution for shifting to a mode of small stimulation energy is employed by changing only the stimulation frequency among the nerve stimulation signal parameters in response to the tachyarrhythmia detection, but it is not obsessed with this mode and it is also possible to shift to a mode of small stimulation energy by changing at least one of stimulation frequency, pulse width, a number of pulses, pulse current, pulse voltage, delay time, rest time and repetition times or a plurality of parameters selected from these for the nerve stimulation signal parameter.

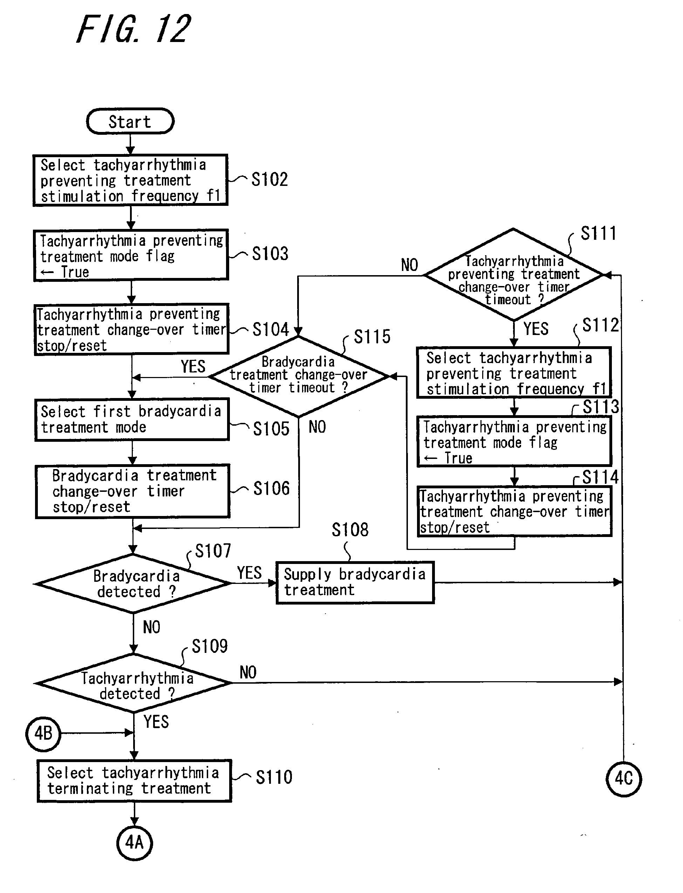

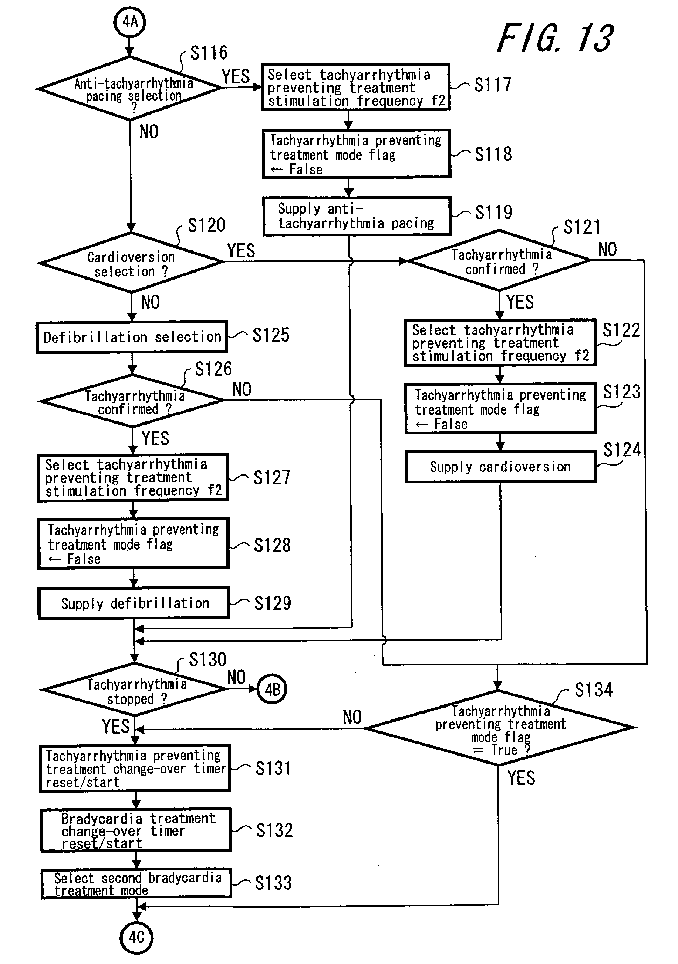

Hereinafter, it will be explained with respect to the operation of the fourth exemplified embodiment of the present invention in detail according to FIG. 12 and FIG. 13.

First, the selection unit 58 of the vagus nerve stimulation and tachyarrhythmia prevention unit 55 selects the stimulation frequency f1 of the first operation mode as an initial value and stimulation of the vagus nerve 41 is carried out by a stimulation waveform having relatively high energy (step S102). Next, "True" is written in the tachyarrhythmia preventing treatment mode flag, which is not shown, of the vagus nerve stimulation and tachyarrhythmia prevention unit 55 (step S103). Then, the tachyarrhythmia preventing treatment change-over timer 20 is stopped/reset in the timing unit 8 (step S104), the first bradycardia treatment mode of the first bradycardia treatment unit 23 is selected by the bradycardia treatment selection unit 28 in the bradycardia treatment unit 9 (step S105) and at the same time, the bradycardia treatment change-over timer 25 is stopped/reset (step S106).

Next, it is judged whether or not a bradycardia was detected in the heart contraction detection unit 2 (step S107) and in a case when a bradycardia was detected, a bradycardia treatment is supplied by the pacing pulse generation unit 10 with respect to the heart 38 (step S108). In a case when a bradycardia was not detected in judgment step S107, it is judged subsequently whether or not a tachyarrhythmia was detected in the tachyarrhythmia detection unit 3 (step S109) and in a case when a tachyarrhythmia was detected, a signal is transmitted to the tachyarrhythmia termination unit 12 and a tachyarrhythmia terminating treatment corresponding to the detected tachyarrhythmia is selected among the anti-tachyarrhythmia pacing, the cardioversion and the defibrillation (step S110). Thereafter, the flow proceeds to 4A of FIG. 13.

In a case when a bradycardia treatment is supplied in step S108 or a tachyarrhythmia was not detected in judgment step S109, it is judged whether or not the tachyarrhythmia preventing treatment change-over timer 20 of the timing unit 8 is timeout (step S111). In a case when the tachyarrhythmia preventing treatment change-over timer 20 is timeout, that is, in a case when the tachyarrhythmia preventing treatment change-over timer 20 made clocking beyond the time stored in change-over time memory unit 21, a signal is transmitted to the selection unit 58 through the second control unit 63 of the control unit 7 and the stimulation frequency f1 of the first operation mode is selected so as to give a relatively strong stimulation to the vagus nerve 41 (step S112) and at the same time, "True" is written in the tachyarrhythmia preventing treatment mode flag of the vagus nerve stimulation and tachyarrhythmia prevention unit 55 (step S113), and subsequently, the tachyarrhythmia preventing treatment change-over timer 20 is stopped/reset (step S114).

Next, in a case when it is judged in judgment step S111 that the tachyarrhythmia preventing treatment change-over timer 20 is not timeout and after the tachyarrhythmia preventing treatment change-over timer 20 was stopped/reset in step S114, it is judged whether or not the bradycardia treatment change-over timer 25 in the bradycardia treatment unit 9 is timeout, that is, whether or not the bradycardia treatment change-over timer 25 made clocking beyond the time stored in the bradycardia treatment change-over time memory unit 26 (step S115).

Then, when it is judged that the bradycardia treatment change-over timer 25 is timeout, the flow returns to the selection of the first bradycardia treatment mode in step S105 and in a case when the bradycardia treatment change-over timer 25 is not timeout, a new bradycardia detection is waited for in judgment step S107.

Next, it will be explained with respect to the operation of the fourth exemplified embodiment of the present invention after the selection of the tachyarrhythmia terminating treatment was carried out in the step S110 (after 4A) according to the flow diagram of FIG. 13.

First, it is judged whether or not an anti-tachyarrhythmia pacing treatment is selected as an anti-tachyarrhythmia treatment (tachyarrhythmia terminating treatment) in the tachyarrhythmia termination unit 12 (step S116). In a case when an anti-tachyarrhythmia pacing treatment is selected, the first control unit 62 of the control unit 7 is activated so as to select the stimulation frequency f2 of the second operation mode in the selection unit 58 of the vagus nerve stimulation and tachyarrhythmia prevention unit 55 (step S117) and at the same time, "False" is written in the tachyarrhythmia preventing treatment mode flag of the vagus nerve stimulation and tachyarrhythmia prevention unit 55 (step S118) and subsequently, the anti-tachyarrhythmia treatment output change-over unit 33 of the tachyarrhythmia termination unit 12 connects the anti-tachyarrhythmia pacing unit 30 and the pacing pulse generation unit 10 and an anti-tachyarrhythmia pacing is supplied with respect to the heart 38 (step S119).

In a case when the anti-tachyarrhythmia pacing is not selected in judgment step S116, it is judged next whether or not a cardioversion treatment is selected (step S120). In a case when the cardioversion treatment is selected, the anti-tachyarrhythmia treatment output change-over unit 33 of the tachyarrhythmia termination unit 12 connects the cardioversion unit 31 and the shock pulse generation unit 11 and charging of a predetermined amount of energy corresponding to the cardioversion treatment is carried out with respect to the capacitor of the shock pulse generation unit 11. It is confirmed whether or not the tachyarrhythmia is continuing in the tachyarrhythmia confirmation unit 4 during the period until the charging to the capacitor is completed (step S121). Then, in a case when it was confirmed that the tachyarrhythmia is continuing, a signal is transmitted from the first control unit 62 of the control unit 7 to the selection unit 58 of the vagus nerve stimulation and tachyarrhythmia prevention unit 55 and the stimulation frequency f2 of the second operation mode is selected by the selection unit 58 (step S122) and at the same time, "False" is written in the tachyarrhythmia preventing treatment mode flag of the vagus nerve stimulation and tachyarrhythmia prevention unit 55 (step S123) and subsequently, a cardioversion treatment by the cardioversion unit 31 which was connected by the anti-tachyarrhythmia treatment output change-over unit 33 is supplied to the heart 38 through the shock pulse generation unit 11 (step S124).

When the anti-tachyarrhythmia pacing treatment is not selected in judgment step S116 and when the cardioversion treatment is not selected in judgment step S120 either, that is, when the defibrillation treatment is selected (step S125), the anti-tachyarrhythmia treatment output change-over unit 33 of the tachyarrhythmia termination unit 12 connects the defibrillation unit 32 and the shock pulse generation unit 11 and charging of a predetermined amount of energy corresponding to the defibrillation treatment is carried out with respect to the capacitor of the shock pulse generation unit 11. It is confirmed whether or not the tachyarrhythmia is continuing in the tachyarrhythmia confirmation unit 4 during the period until the charging to the capacitor is completed (step S126). Then, in a case when it was confirmed that the tachyarrhythmia is continuing, a signal is transmitted to the selection unit 58 of the vagus nerve stimulation and tacharrhythmia prevention unit 55 by the first control unit 62 of the control unit 7 and the stimulation frequency f2 of the second operation mode is selected (step S127) and at the same time, "False" is written in the tachyarrhythmia preventing treatment mode flag of the vagus nerve stimulation and tachyarrhythmia prevention unit 55 (step S128) and subsequently, a defibrillation treatment by the defibrillation unit 32 which was connected by the anti-tachyarrhythmia treatment output change-over unit 33 is supplied to the heart 38 through the shock pulse generation unit 11 (step S129).

Next, after the anti-tachyarrhythmia pacing treatment of step S119, the cardioversion treatment of step S124 or the defibrillation treatment of step S129 is supplied with respect to the heart 38, it is judged in the tachyarrhythmia termination confirmation unit 5 whether or not the tachyarrhythmia is terminated (step S130). Then, in a case when it is confirmed that the tachyarrhythmia terminated, the tachyarrhythmia preventing treatment change-over timer 20 of the timing unit 8 is reset/started (step S131) and subsequently, the bradycardia treatment change-over timer 25 of the bradycardia treatment unit 9 is reset/started (step S132) and at the same time, the second bradycardia treatment mode of the second bradycardia treatment unit 24 is selected by the bradycardia treatment selection unit 28 (step S133) and the flow returns to judgment step S111 of FIG. 12.

In a case when the tachyarrhythmia termination is not confirmed in judgment step S130, the flow returns to step S110 of FIG. 12 and selection of the tachyarrhythmia terminating treatment is again carried out, but in this selection of the tachyarrhythmia terminating treatment, augmentation of the applied energy, change of the tacharrhythmia terminating treatment method or the like is carried out in a stepwise manner according to a predetermined treatment plan.

In a case when continuation of the tachyarrhythmia is not confirmed in judgment step S121 and judgment step S126, that is, in a case when the tachyarrhythmia terminated spontaneously amid the charging to the capacitor of the shock pulse generation unit 11, releasing of charging stop, internal discharge of the charging energy and connection between the cardioversion unit 31 of the tachyarrhythmia termination unit 12 or the defibrillation unit 32 and the shock pulse generation unit 11 are carried out and at the same time, it is judged whether or not the tachyarrhythmia preventing treatment mode flag of the vagus nerve stimulation and tachyarrhythmia prevention unit 55 is "True" (step S134). Then, in a case when the tachyarrhythmia preventing treatment mode flag is "True", that is, when the stimulation frequency f1 of the first operation mode is selected in the tachyarrhythmia preventing treatment of the vagus nerve stimulation and tachyarrhythmia prevention unit 55, the flow returns to judgment step S111 of FIG. 12 and in a case when the tachyarrhythmia preventing treatment mode flag is not "True", that is, when the stimulation frequency f2 of the second operation mode is selected in the tachyarrhythmia preventing treatment of the vagus nerve stimulation and tachyarrhythmia prevention unit 55, the tachyarrhythmia preventing treatment program of the tachyarrhythmia prevention unit 6 is not activated, it is assumed that the possibility that the heart is still in an unstable situation is high so that processes subsequent to step S131, which are sequences after the tachyarrhythmia termination are carried out through the tachyarrhythmia termination confirmation unit 5.

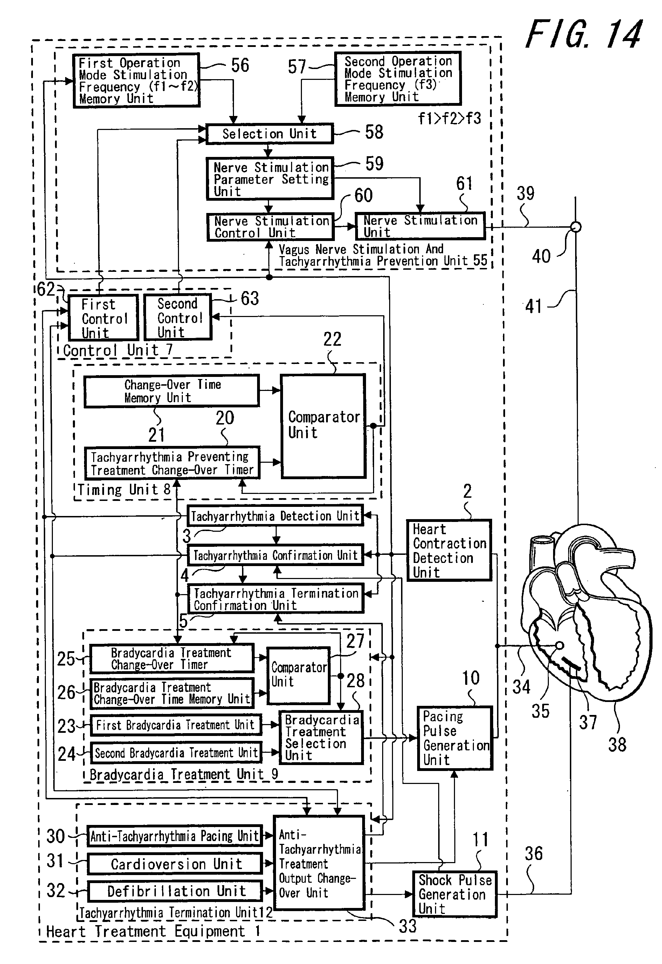

FIG. 14 is a block constitutional diagram showing a fifth exemplified embodiment of the heart treatment equipment according to the present invention. The constitution of the fifth exemplified embodiment of the present invention is approximately equal to the constitution of the fourth exemplified embodiment of the present invention shown in FIG. 11 and the constitutional difference thereof lies in that while the first operation mode stimulation frequency is fixed to f1 in the fourth exemplified embodiment, the stimulation frequency of the first operation mode in the fifth exemplified embodiment is made to vary in a range of f1 to f2 (f1 > f2) as to the output of the heart contraction detection unit 2. Also, the stimulation frequency f3 of the second operation mode is made to be a further lower frequency than f2. Constitutions other than the aforesaid constitutional portions are same as those of the block constitutional diagrams of FIG. 11, so that the same reference numerals are put thereon and the explanation thereof will be omitted.

It should be noted in this example that a constitution for shifting to a mode of small stimulation energy is employed by changing only the stimulation frequency among the nerve stimulation signal parameters in response to the tachyarrhythmia detection, but it is not obsessed with this mode and it is also possible to shift to a mode of small stimulation energy by changing at least one of stimulation frequency, pulse width, a number of pulses, pulse current, pulse voltage, delay time, rest time and repetition times or a plurality of parameters selected from these for the nerve stimulation signal parameter.

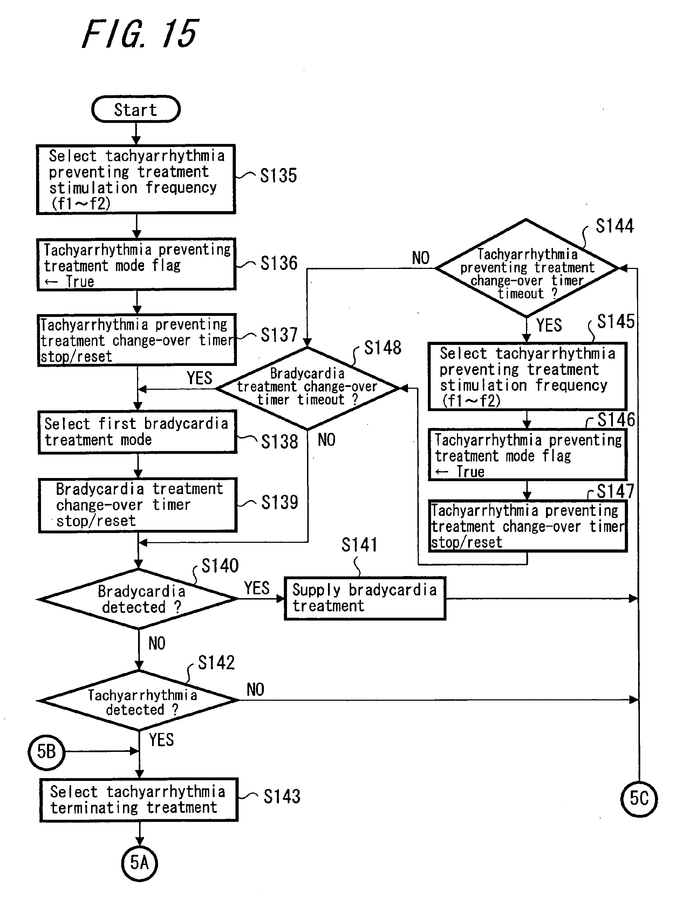

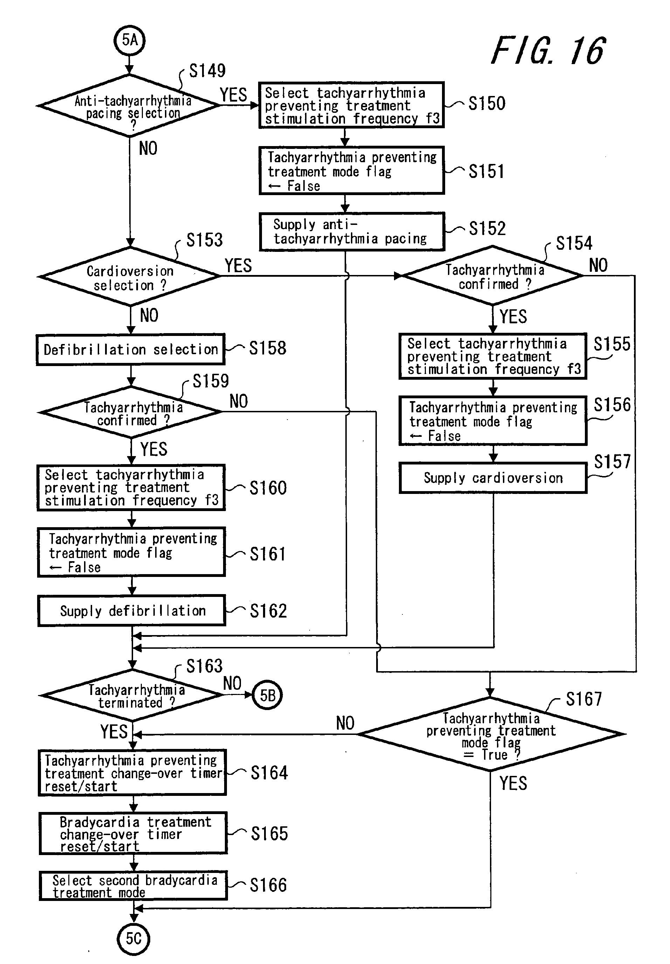

Hereinafter, it will be explained with respect to the operation of the fifth exemplified embodiment of the present invention in detail according to FIG. 15 and FIG. 16. There are many repetitive portions in this explanation with respect to the explanations of the flow diagrams in FIG. 13 and FIG. 14, but reference numerals of respective processes therein are changed, so that they will be explained repetitively.

First, the selection unit 58 of the vagus nerve stimulation and tachyarrhythmia prevention unit 55 selects the stimulation frequency (f1 to f2) of the first operation mode as an initial value and stimulation of the vagus nerve 41 is carried out by a stimulation waveform having relatively high energy in this frequency range (step S135). Next, "True" is written in the tachyarrhythmia preventing treatment mode flag, which is not shown, of the vagus nerve stimulation and tachyarrhythmia prevention unit 55 (step S136). Then, the tachyarrhythmia preventing treatment change-over timer 20 is stopped/reset in the timing unit 8 (step S137), the first bradycardia treatment mode of the first bradycardia treatment unit 23 is selected by the bradycardia treatment selection unit 28 in the bradycardia treatment unit 9 (step S138) and at the same time, the bradycardia treatment change-over timer 25 is stopped/reset (step S139).

Next, it is judged whether or not a bradycardia was detected in the heart contraction detection unit 2 (step S140) and in a case when a bradycardia was detected, a bradycardia treatment is supplied by the pacing pulse generation unit 10 with respect to the heart 38 (step S141). In a case when a bradycardia was not detected in judgment step S140, it is judged subsequently whether or not a tachyarrhythmia was detected in the tachyarrhythmia detection unit 3 (step S142) and in a case when a tachyarrhythmia was detected, a signal is transmitted to the tachyarrhythmia termination unit 12 and a tachyarrhythmia terminating treatment corresponding to the detected tachyarrhythmia is selected among the anti-tachyarrhythmia pacing, the cardioversion and the defibrillation (step S143). Thereafter, the flow proceeds to 5A of FIG. 16.

In a case when a bradycardia treatment is supplied in step S141 or a tachyarrhythmia was not detected in judgment step S142, it is judged whether or not the tachyarrhythmia preventing treatment change-over timer 20 of the timing unit 8 is timeout (step S144). In a case when the tachyarrhythmia preventing treatment change-over timer 20 is timeout, that is, in a case when the tachyarrhythmia preventing treatment change-over timer 20 made clocking beyond the time stored in change-over time memory unit 21, a signal is transmitted to the selection unit 58 through the second control unit 63 of the control unit 7 and the stimulation frequency (f1 to f2) of the first operation mode is selected so as to give a relatively strong stimulation to the vagus nerve 41 (step S145) and at the same time, "True" is written in the tachyarrhythmia preventing treatment mode flag of the vagus nerve stimulation and tachyarrhythmia prevention unit 55 (step S146), and subsequently, the tachyarrhythmia preventing treatment change-over timer 20 is stopped/reset (step S147).

Next, in a case when it is judged in judgment step S144 that the tachyarrhythmia preventing treatment change-over timer 20 is not timeout and after the tachyarrhythmia preventing treatment change-over timer 20 was stopped/reset in step S147, it is judged whether or not the bradycardia treatment change-over timer 25 in the bradycardia treatment unit 9 is timeout, that is, whether or not the bradycardia treatment change-over timer 25 made clocking beyond the time stored in the bradycardia treatment change-over time memory unit 26 (step S148).

Then, when it is judged that the bradycardia treatment change-over timer 25 is timeout, the flow returns to the selection of the first bradycardia treatment mode in step S138 and in a case when the bradycardia treatment change-over timer 25 is not timeout, a new bradycardia detection is waited for in judgment step S140.

Next, it will be explained with respect to the operation of the fifth exemplified embodiment of the present invention after the selection of the tachyarrhythmia terminating treatment was carried out in the step S143 (after 5A) according to the flow diagram of FIG. 16.

First, it is judged whether or not an anti-tachyarrhythmia pacing treatment is selected as an anti-tachyarrhythmia treatment (tachyarrhythmia terminating treatment) in the tachyarrhythmia termination unit 12 (step S149). In a case when an anti-tachyarrhythmia pacing treatment is selected, the first control unit 62 of the control unit 7 is activated so as to select the stimulation frequency f3 of the second operation mode in the selection unit 58 of the vagus nerve stimulation and tachyarrhythmia prevention unit 55 (step S150) and at the same time, "False" is written in the tachyarrhythmia preventing treatment mode flag of the vagus nerve stimulation and tachyarrhythmia prevention unit 55 (step S151) and subsequently, the anti-tachyarrhythmia treatment output change-over unit 33 of the tachyarrhythmia termination unit 12 connects the anti-tachyarrhythmia pacing unit 30 and the pacing pulse generation unit 10 and an anti-tachyarrhythmia pacing is supplied with respect to the heart 38 (step S152).

In a case when the anti-tachyarrhythmia pacing is not selected in judgment step S149, it is judged next whether or not a cardioversion treatment is selected (step S153). In a case when the cardioversion treatment is selected, the anti-tachyarrhythmia treatment output change-over unit 33 of the tachyarrhythmia termination unit 12 connects the cardioversion unit 31 and the shock pulse generation unit 11 and charging of a predetermined amount of energy corresponding to the cardioversion treatment is carried out with respect to the capacitor of the shock pulse generation unit 11. It is confirmed whether or not the tachyarrhythmia is continuing in the tachyarrhythmia confirmation unit 4 during the period until the charging to the capacitor is completed (step S154). Then, in a case when it was confirmed that the tachyarrhythmia is continuing, a signal is transmitted from the first control unit 62 of the control unit 7 to the selection unit 58 of the vagus nerve stimulation and tachyarrhythmia prevention unit 55 and the stimulation frequency f3 of the second operation mode is selected by the selection unit 58 (step S155) and at the same time, "False" is written in the tachyarrhythmia preventing treatment mode flag of the vagus nerve stimulation and tachyarrhythmia prevention unit 55 (step S156) and subsequently, a cardioversion treatment by the cardioversion unit 31 which was connected by the anti-tachyarrhythmia treatment output change-over unit 33 is supplied to the heart 38 through the shock pulse generation unit 11 (step S157).

When the anti-tachyarrhythmia pacing treatment is not selected in judgment step S149 and when the cardioversion treatment is not selected in judgment step S153 either, that is, when the defibrillation treatment is selected (step S158), the anti-tachyarrhythmia treatment output change-over unit 33 of the tachyarrhythmia termination unit 12 connects the defibrillation unit 32 and the shock pulse generation unit 11 and charging of a predetermined amount of energy corresponding to the defibrillation treatment is carried out with respect to the capacitor of the shock pulse generation unit 11. It is confirmed whether or not the tachyarrhythmia is continuing in the tachyarrhythmia confirmation unit 4 during the period until the charging to the capacitor is completed (step S159). Then, in a case when it was confirmed that the tachyarrhythmia is continuing, a signal is transmitted to the selection unit 58 of the vagus nerve stimulation and tachyarrhythmia prevention unit 55 by the first control unit 62 of the control unit 7 and the stimulation frequency f3 of the second operation mode is selected (step S160) and at the same time, "False" is written in the tachyarrhythmia preventing treatment mode flag of the vagus nerve stimulation and tachyarrhythmia prevention unit 55 (step S161) and subsequently, a defibrillation treatment by the defibrillation unit 32 which was connected by the anti-tachyarrhythmia treatment output change-over unit 33 is supplied to the heart 38 through the shock pulse generation unit 11 (step S162).

Next, after the anti-tachyarrhythmia pacing treatment of step S152, the cardioversion treatment of step S157 or the defibrillation treatment of step S162 is supplied with respect to the heart 38, it is judged in the tachyarrhythmia termination confirmation unit 5 whether or not the tachyarrhythmia is terminated (step S163). Then, in a case when it is confirmed that the tachyarrhythmia terminated, the tachyarrhythmia preventing treatment change-over timer 20 of the timing unit 8 is reset/started (step S164) and subsequently, the bradycardia treatment change-over timer 25 of the bradycardia treatment unit 9 is reset/started (step S165) and at the same time, the second bradycardia treatment mode of the second bradycardia treatment unit 24 is selected by the bradycardia treatment selection unit 28 (step S166) and the flow returns to judgment step S144 of FIG. 15.

In a case when the tachyarrhythmia termination is not confirmed in judgment step S163, the flow returns to step S143 of FIG. 15 and selection of the tachyarrhythmia terminating treatment is again carried out, but in this selection of the tachyarrhythmia terminating treatment, augmentation of the applied energy, change of the tachyarrhythmia terminating treatment method or the like is carried out in a stepwise manner according to a predetermined treatment plan.

In a case when continuation of the tachyarrhythmia is not confirmed in judgment step S154 and judgment step S159, that is, in a case when the tachyarrhythmia terminated spontaneously amid the charging to the capacitor of the shock pulse generation unit 11, releasing of charging stop, internal discharge of the charging energy and connection between the cardioversion unit 31 of the tachyarrhythmia termination unit 12 or the defibrillation unit 32 and the shock pulse generation unit 11 are carried out and at the same time, it is judged whether or not the tachyarrhythmia preventing treatment mode flag of the vagus nerve stimulation and tachyarrhythmia prevention unit 55 is "True" (step S167). Then, in a case when the tachyarrhythmia preventing treatment mode flag is "True", that is, when the stimulation frequency (f1 to f2) of the first operation mode is selected in the tachyarrhythmia preventing treatment of the vagus nerve stimulation and tachyarrhythmia prevention unit 55, the flow returns to judgment step S144 of FIG. 15 and in a case when the tachyarrhythmia preventing treatment mode flag is not "True", that is, when the stimulation frequency f3 of the second operation mode is selected in the tachyarrhythmia preventing treatment of the vagus nerve stimulation and tachyarrhythmia prevention unit 55, the tachyarrhythmia preventing treatment program of the tachyarrhythmia prevention unit 6 is not activated, it is assumed that the possibility that the heart is still in an unstable situation is high so that processes subsequent to step S164, which are sequences after the tachyarrhythmia termination are carried out through the tachyarrhythmia termination confirmation unit 5.

As described above, the explanations of the constitutions and the operations of the first to the fifth exemplified embodiments according to the present invention were carried out, but it is needless to say that the present invention includes exemplified embodiments other than aforesaid embodiments without being obsessed with those exemplified embodiments and in a scope without departing from the description of the claims.

As explained in the above, according to the heart treatment equipment of the present invention, there is provided with a control structure for reducing or stopping the activation current with respect to the vagus nerve or a repression current with respect to the sympathetic nerve after an arrhythmia treatment such as an electroshock to the heart by the cardioversion treatment, the defibrillation treatment or the like and it is constituted such that the tachyarrhythmia preventing treatment is made to cancel or it is made possible to shift to a mode in which the nerve stimulation energy is smaller in response to the confirmation of the tachyarrhythmia detection or the tachyarrhythmia continuation, so that it is possible to solve a problem with deterioration of hemodynamics, recurrence of a fatal arrhythmia, induction of supraventricular arrhythmia such as atrial fibrillation or the like and the like.

### DESCRIPTION OF REFERENCE NUMERALS

- 1: heart treatment equipment
- 2: heart contraction detection unit
- 3: tachyarrhythmia detection unit
- 4: tachyarrhythmia confirmation unit
- 5: tachyarrhythmia termination confirmation unit
- 6: ·tachyarrhythmia prevention unit
- 7: control unit
- 8: timing unit
- 9: bradycardia treatment unit
- 10: pacing pulse generation unit
- 11: shock pulse generation unit
- 12: tachyarrhythmia termination unit
- 13: sympathetic nerve stimulation unit
- 14: parasympathetic nerve stimulation unit
- 15: blocking stimulation unit
- 16: sub-threshold stimulation unit
- 17: activating stimulation unit
- 18: deactivation unit
- 19: activation unit
- 30: anti-tachyarrhythmia pacing unit
- 31: cardioversion unit
- 32: defibrillation unit
- 38: heart
- 41: vagus nerve
- 44: ganglion stellatum

## Claims

1. Heart treatment equipment comprising:
tachyarrhythmia prevention means for preventing occurrence of tachyarrhythmia;
tachyarrhythmia detection means for detecting said occurrence of tachyarrhythmia;
tachyarrhythmia termination means supplying anti-tachyarrhythmia treatment for terminating said tachyarrhythmia in response to said tachyarrhythmia detection means; and control means for connecting said tachyarrhythmia prevention means and said tachyarrhythmia detection means,
wherein said control means includes means for deactivating said tachyarrhythmia prevention means in its operation in response to said tachyarrhythmia detection means.

2. Heart treatment equipment comprising:
tachyarrhythmia prevention means for preventing occurrence of tachyarrhythmia;
tachyarrhythmia detection means for detecting said occurrence of tachyarrhythmia;
tachyarrhythmia confirmation means for confirming that said tachyarrhythmia continues in response to said tachyarrhythmia detection means;
tachyarrhythmia termination means supplying anti-tachyarrhythmia treatment for terminating said tachyarrhythmia in response to said tachyarrhythmia confirmation means; and control means for connecting said tachyarrhythmia prevention means and said tachyarrhythmia confirmation means,
wherein said control means includes means for deactivating said tachyarrhythmia prevention means in its operation in response to said tachyarrhythmia confirmation means.

3. Heart treatment equipment according to claim 1 or claim 2, wherein said tachyarrhythmia prevention means includes nerve stimulation means.

4. Heart treatment equipment according to claim 3, wherein said nerve stimulation means is means for stimulating a parasympathetic nerve.

5. Heart treatment equipment according to claim 4, wherein said parasympathetic nerve stimulation is a stimulation for activating excitement of the parasympathetic nerve.

6. Heart treatment equipment according to claim 4 or claim 5, wherein said parasympathetic nerve is a vagus nerve.

7. Heart treatment equipment according to claim 3, wherein said nerve stimulation means is means for stimulating a sympathetic nerve.

8. Heart treatment equipment according to claim 7, wherein said sympathetic nerve stimulation is a stimulation having a level which does not increase heart rate or blood pressure.

9. Heart treatment equipment according to claim 7, wherein said sympathetic nerve stimulation is a stimulation for blocking transmission of the sympathetic nerve excitement.

10. Heart treatment equipment according to any one of claims 7 to 9, wherein said sympathetic nerve is a ganglion stellatum.

11. Heart treatment equipment according to claim 1 or claim 2, wherein said anti-tachyarrhythmia treatment which said tachyarrhythmia termination means supplies includes at least one of anti-tachyarrhythmia pacing, cardioversion and defibrillation.

12. Heart treatment equipment according to claim 1 or claim 2 further comprising bradycardia detection means for detecting occurrence of bradycardia; and bradycardia treatment means for generating a heart stimulation pulse in response to said bradycardia detection means.

13. Heart treatment equipment according to claim 1 or claim 2, wherein said control means further includes means for activating said tachyarrhythmia prevention means subsequent to the deactivation of said tachyarrhythmia prevention means.

14. Heart treatment equipment according to claim 13 further comprising timing means for clocking a predetermined time, wherein said activation means activates said tachyarrhythmia prevention means in response to said timing means.

15. Heart treatment equipment according to claim 14, wherein said timing means sets a clocking time in conformity with said anti-tachyarrhythmia treatment in response to the supply Of said anti-tachyarrhythmia treatment of said tachyarrhythmia termination means.

16. Heart treatment equipment according to claim 13 further comprising heart rate measuring means for measuring heartbeats, wherein said activation means activates said tachyarrhythmia prevention means in response to said heart rate measuring means.

17. Heart treatment equipment according to claim 16, wherein said activation means activates said tachyarrhythmia prevention means in a case when the heart rate which said heart rate measuring means measured exceeds a predetermined threshold.

18. Heart treatment equipment according to claim 14 or claim 15 further comprising tachyarrhythmia termination confirmation means for confirming whether or not said tachyarrhythmia was terminated by said tachyarrhythmia termination means, wherein said timing means starts clocking of said predetermined time in response to said tachyarrhythmia termination confirmation means.

19. Heart treatment equipment according to claim 16 or claim 17 further comprising tachyarrhythmia termination confirmation means for confirming whether or not said tachyarrhythmia was terminated by said tachyarrhythmia termination means, wherein said activation according to said heart rate measuring means is carried out in response to said tachyarrhythmia termination confirmation means.

20. Heart treatment equipment comprising:
tachyarrhythmia prevention means for preventing occurrence of tachyarrhythmia by a nerve stimulation;
tachyarrhythmia detection means for detecting said occurrence of tachyarrhythmia;
tachyarrhythmia termination means supplying anti-tachyarrhythmia treatment for terminating said tachyarrhythmia in response to said tachyarrhythmia detection means; and control means for connecting said tachyarrhythmia prevention means and said tachyarrhythmia detection means,
wherein said nerve stimulation by said tachyarrhythmia prevention means operates at least in a first operation mode and a second operation mode, nerve stimulation energy of said second operation mode is set to be lower than nerve stimulation energy of said first operation mode and said control means includes first control means for operating said tachyarrhythmia prevention means in said second operation mode in response to said tachyarrhythmia detection means.

21. Heart treatment equipment comprising:
tachyarrhythmia prevention means for preventing occurrence of tachyarrhythmia by a nerve stimulation;
tachyarrhythmia detection means for detecting said occurrence of tachyarrhythmia;
tachyarrhythmia confirmation means for confirming that said tachyarrhythmia continues in response to said tachyarrhythmia detection means;
tachyarrhythmia termination means supplying anti-tachyarrhythmia treatment for terminating said tachyarrhythmia in response to tachyarrhythmia confirmation means; and control means for connecting said tachyarrhythmia prevention means and said tachyarrhythmia confirmation means,
wherein said nerve stimulation by said tachyarrhythmia prevention means operates at least in a first operation mode and a second operation mode, nerve stimulation energy of said second operation mode is set to be lower than nerve stimulation energy of said first operation mode and said control means includes first control means for operating said tachyarrhythmia prevention means in said second operation mode in response to said tachyarrhythmia confirmation means.

22. Heart treatment equipment according to claim 20 or claim 21, wherein said nerve stimulation means is means for stimulating a parasympathetic nerve.

23. Heart treatment equipment according to claim 22, wherein said parasympathetic nerve stimulation is a stimulation for activating excitement of the parasympathetic nerve.

24. Heart treatment equipment according to claim 22 or claim 23, wherein said parasympathetic nerve is a vagus nerve.

25. Heart treatment equipment according to claim 20 or claim 21, wherein said nerve stimulation means is means for stimulating a sympathetic nerve.

26. Heart treatment equipment according to claim 25, wherein said sympathetic nerve stimulation is a stimulation having a level which does not increase heart rate or blood pressure.

27. Heart treatment equipment according to claim 25, wherein said sympathetic nerve stimulation is a stimulation for blocking transmission of the sympathetic nerve excitement.

28. Heart treatment equipment according to any one of claims 25 to 27, wherein said sympathetic nerve is a ganglion stellatum.

29. Heart treatment equipment according to claim 20 or claim 21, wherein said anti-tachyarrhythmia treatment which said tachyarrhythmia termination means supplies includes at least one of anti-tachyarrhythmia pacing, cardioversion and defibrillation.

30. Heart treatment equipment according to claim 20 or claim 21 further comprising bradycardia detection means for detecting occurrence of bradycardia; and bradycardia treatment means for generating a heart stimulation pulse in response to said bradycardia detection means.

31. Heart treatment equipment according to claim 20 or claim 21, wherein said nerve stimulation energy setting is carried out by adjusting a nerve stimulation signal parameter of said nerve stimulation.

32. Heart treatment equipment according to claim 31, wherein said nerve stimulation signal parameter is at least one of stimulation frequency, pulse width, a number of pulses, pulse current, pulse voltage, delay time, rest time and repetition times or a combination of a plurality of ones selected from these.

33. Heart treatment equipment according to claim 20 or claim 21, wherein said control means further includes second control means for operating said tachyarrhythmia prevention means in said first operation mode subsequently to the operation in said second operation mode.

34. Heart treatment equipment according to claim 33 further comprising timing means for clocking a predetermined time, wherein said second control means operates in response to said timing means.

35. Heart treatment equipment according to claim 34, wherein said timing means sets a clocking time in conformity with said anti-tachyarrhythmia treatment in response to the supply of said anti-tachyarrhythmia treatment of said tachyarrhythmia termination means.

36. Heart treatment equipment according to claim 33 further comprising heart rate measuring means for measuring heartbeats, wherein said second control means operates in response to said heart rate measuring means.

37. Heart treatment equipment according to claim 36, wherein said second control means operates in a case when the heart rate which said heart rate measuring means measured exceeds a predetermined threshold.

38. Heart treatment equipment according to claim 34 or claim 35 further comprising tachyarrhythmia termination confirmation means for confirming whether or not said tachyarrhythmia is terminated by said tachyarrhythmia termination means, wherein said timing means starts clocking of said predetermined time in response to said tachyarrhythmia termination confirmation means.

39. Heart treatment equipment according to claim 36 or claim 37 further comprising tachyarrhythmia termination confirmation means for confirming whether or not said tachyarrhythmia is terminated by said tachyarrhythmia termination means, wherein the operation of said second control means according to said heart rate measuring means is carried out in response to said tachyarrhythmia termination confirmation means.

40. A heart treatment method comprising:
an activation step for observing heart activity and for activating a tachyarrhythmia prevention program which carries out a nerve stimulation if necessary;
a tachyarrhythmia observation step for observing said occurrence of tachyarrhythmia; and
a step for carrying out anti-tachyarrhythmia treatment for terminating said tachyarrhythmia according to said tachyarrhythmia detection by said tachyarrhythmia observation step and at the same time for deactivating said tachyarrhythmia prevention program.

41. A heart treatment method comprising:
an activation step for observing heart activity and for activating a tachyarrhythmia prevention program which carries out a nerve stimulation if necessary;
a tachyarrhythmia observation step for observing said occurrence of tachyarrhythmia;
a tachyarrhythmia confirmation step for confirming that said tachyarrhythmia continues in response to said tachyarrhythmia detection by said tachyarrhythmia observation step; and
a step for carrying out anti-tachyarrhythmia treatment for terminating said tachyarrhythmia according to the confirmation of said tachyarrhythmia continuation and at the same time for deactivating said tachyarrhythmia prevention program.

42. Heart treatment method according to claim 40 or claim 41, wherein said nerve stimulation means is means for stimulating a parasympathetic nerve.

43. Heart treatment method according to claim 42, wherein said parasympathetic nerve stimulation is stimulation for activating excitement of the parasympathetic nerve.

44. Heart treatment method according to claim 42 or claim 43, wherein said parasympathetic nerve is a vagus nerve.

45. Heart treatment method according to claim 40 or claim 41, wherein said nerve stimulation means is means for stimulating a sympathetic nerve.

46. Heart treatment method according to claim 45, wherein said sympathetic nerve stimulation is a stimulation having a level which does not increase heart rate or blood pressure.

47. Heart treatment method according to claim 45, wherein said sympathetic nerve stimulation is a stimulation for blocking transmission of the sympathetic nerve excitement.

48. Heart treatment method according to claim 45, wherein said sympathetic nerve is a ganglion stellatum.

49. Heart treatment method according to claim 40 or claim 41, wherein said anti-tachyarrhythmia treatment includes at least one of anti-tachyarrhythmia pacing, cardioversion and defibrillation.

50. Heart treatment method according to claim 40 or claim 41 further comprising bradycardia detection step for detecting occurrence of bradycardia and for generating a heart stimulation pulse in response to the bradycardia detection.

51. Heart treatment method according to claim 40 or claim 41 further comprising a re-activation step for again activating said tachyarrhythmia prevention program deactivated by a step for deactivating the tachyarrhythmia prevention program.

52. Heart treatment method according to claim 51, wherein the re-activation of said tachyarrhythmia prevention program is carried out after a predetermined time elapsed after said tachyarrhythmia prevention program deactivated.

53. Heart treatment method according to claim 52, wherein said predetermined time is set to a time in conformity with said anti-tachyarrhythmia treatment.

54. Heart treatment method according to claim 51, wherein the re-activation of said tachyarrhythmia prevention program is carried out according to heartbeats measurement.

55. Heart treatment method according to claim 54, wherein the re-activation of said tachyarrhythmia prevention program is carried out in a case when the heart rate obtained by said measurement exceeds a predetermined threshold.

56. Heart treatment method according to claim 52, wherein said predetermined time clocking starts after said tachyarrhythmia was confirmed to be terminated by said anti-tachyarrhythmia treatment.

57. Heart treatment method according to claim 54, wherein said heartbeats measurement starts after said tachyarrhythmia was confirmed to be terminated by said anti-tachyarrhythmia treatment.

58. A heart treatment method comprising:
an activation step for observing heart activity and for activating a tachyarrhythmia prevention program which carries out a nerve stimulation in a first operation mode if necessary;
a tachyarrhythmia observation step for observing said occurrence of tachyarrhythmia; and
a step for carrying out anti-tachyarrhythmia treatment for terminating said tachyarrhythmia according to said tachyarrhythmia detection by said tachyarrhythmia observation step and at the same time for changing said tachyarrhythmia prevention program so as to carry out a nerve stimulation in a second operation mode which has smaller nerve stimulation energy than nerve stimulation energy of the first operation mode.

59. A heart treatment method comprising:
an activation step for observing heart activity and for activating a tachyarrhythmia prevention program which carries out a nerve stimulation in a first operation mode if necessary;
a tachyarrhythmia observation step for observing said occurrence of tachyarrhythmia;
tachyarrhythmia confirmation step for confirming that said tachyarrhythmia continues in response to said tachyarrhythmia detection by said tachyarrhythmia observation step; and
a step for carrying out anti-tachyarrhythmia treatment for terminating said tachyarrhythmia according to the confirmation of said tachyarrhythmia continuation and at the same time for changing said tachyarrhythmia prevention program so as to carry out a nerve stimulation in a second operation mode which has smaller nerve stimulation energy than nerve stimulation energy of the first operation mode.

60. Heart treatment method according to claim 58 or claim 59, wherein said nerve stimulation means is means for stimulating a parasympathetic nerve.

61. Heart treatment method according to claim 60, wherein said parasympathetic nerve stimulation is stimulation for activating excitement of the parasympathetic nerve.

62. Heart treatment method according to claim 60, wherein said parasympathetic nerve is a vagus nerve.

63. Heart treatment method according to claim 58 or claim 59, wherein said nerve stimulation means is means for stimulating a sympathetic nerve.

64. Heart treatment method according to claim 63, wherein said sympathetic nerve stimulation is a stimulation having a level which does not increase heart rate or blood pressure.

65. Heart treatment method according to claim 63, wherein said sympathetic nerve stimulation is a stimulation for blocking transmission of the sympathetic nerve excitement.

66. Heart treatment method according to claim 63, wherein said sympathetic nerve is a ganglion stellatum.

67. Heart treatment method according to claim 58 or claim 59, wherein said anti-tachyarrhythmia treatment includes at least one of anti-tachyarrhythmia pacing, cardioversion and defibrillation.

68. Heart treatment method according to claim 58 or claim 59 further comprising bradycardia detection step for detecting occurrence of bradycardia and for generating a heart stimulation pulse in response to the bradycardia detection.

69. Heart treatment method according to claim 58 or claim 59, wherein said nerve stimulation energy setting is carried out by adjusting a nerve stimulation signal parameter of said nerve stimulation.

70. Heart treatment method according to claim 69, wherein said nerve stimulation signal parameter is at least one of stimulation frequency, pulse width, a number of pulses, pulse current, pulse voltage, delay time, rest time and repetition times or a combination of a plurality of ones selected from these.

71. Heart treatment method according to claim 58 or claim 59, wherein said nerve stimulation operated in said second operation mode is changed-over to said first operation mode.

72. Heart treatment method according to claim 71, wherein the change-over to said first operation mode is carried out after operating in said second operation mode for a predetermined time.

73. Heart treatment method according to claim 72, wherein said predetermined time is set to a time in conformity with said anti-tachyarrhythmia treatment.

74. Heart treatment method according to claim 71, wherein the change-over to said first operation mode is carried out according to heartbeats measurement.

75. Heart treatment method according to claim 74, wherein the change-over to said first operation mode is carried out in a case when said measured heart rate exceeds a predetermined threshold.

76. Heart treatment method according to claim 72, wherein said predetermined time clocking starts after said tachyarrhythmia was confirmed to be terminated by said anti-tachyarrhythmia treatment.

77. Heart treatment method according to claim 74, wherein said heartbeat measurement starts after said tachyarrhythmia was confirmed to be terminated by said anti-tachyarrhythmia treatment.
